# EUROPEAN PATENT APPLICATION

(11) **EP 3 693 380 A1**
(43) Date of publication of application: **12.08.2020**
(21) Application number: 19156552.2
(22) Date of filing: 11.02.2019
(51) Int. Cl.: C07K 14/47, C07K 14/705, C12N 15/113, A61P 35/00, A61P 19/08, A61K 39/395, A61K 31/7088

(54) **DUAL INHIBITION OF PLEXIN-B1 AND PLEXIN-B2**

(71) Applicant: Max-Planck-Gesellschaft zur Förderung der Wissenschaften e.V., 80539 München (DE); Philipps Universität Marburg, 35037 Marburg (DE)
(72) Inventor: The designation of the inventor has not yet been filed
(74) Representative: Vossius & Partner Patentanwälte Rechtsanwälte mbB

(57) **Abstract**

The present invention relates to a composition comprising an inhibitor of plexin-B1 and an inhibitor of plexin-B2, wherein the inhibitor of plexin-B1 and/or the inhibitor of plexin-B2 is/are preferably selected from (i) an inhibitor of the nucleic acid molecule encoding plexin-B1 and/or plexin-B2 selected from a small molecule, an aptamer, a siRNA, a shRNA, a miRNA, a morpholino, a ribozyme, an antisense nucleic acid molecule, a CRISPR-Cas9-based construct, a CRISPR-Cpf1-based construct, a meganuclease, a zinc finger nuclease, and a transcription activator-like (TAL) effector (TALE) nuclease, and/or (ii) an inhibitor of the plexin-B1 and/or plexin-B2 protein selected from a small molecule, an antibody or antibody mimetic, an aptamer, wherein the antibody mimetic is preferably selected from affibodies, adnectins, anticalins, DARPins, avimers, nanofitins, affilins, Kunitz domain peptides and Fynomers®.

SEQUENCE LISTING

<110> Max-Planck-Gesellschaft zur Förderung der Wissenschaften e.V.
      Philipps-Universität Marburg

<120> Dual inhibition of plexin-B1 and plexin-B2

<130> AC1219 EP

<160> 20

<170> BiSSAP 1.3

<210> 1
<211> 6408
<212> DNA
<213> Homo sapiens

<220>
<223> Plexin-B1, human, cDNA (coding sequence)

<400> 1
atgcctgctc tgggcccagc tcttctccag gctctctggg ccgggtgggt cctcaccctc      60

cagccccttc caccaactgc attcactccc aatggcacgt atctgcagca cctggcaagg     120

gaccccacct caggcaccct ctacctgggg gctaccaact tcctgttcca gctgagccct     180

gggctgcagc tggaggccac agtgtccacc ggccctgtgc tagacagcag ggactgcctg     240

ccacctgtga tgcctgatga gtgcccccag gcccagccta ccaacaaccc gaatcagctg     300

ctcctggtga gcccaggggc cctggtggta tgcgggagcg tgcaccaggg ggtctgtgaa     360

cagcggcgcc tggggcagct cgagcagctg ctgctgcggc cagagcggcc tggggacaca     420

caatatgtgg ctgccaatga tcctgcggtc agcacggtgg ggctggtagc ccagggcttg     480

gcaggggagc ccctcctgtt tgtggggcga ggatacacca gcaggggtgt ggggggtggc      540

attccaccca tcacaacccg ggccctgtgg ccgcccgacc cccaagctgc cttctcctat     600

gaggagacag ccaagctggc agtgggccgc ctctccgagt acagccacca cttcgtgagt     660

gcctttgcac gtggggccag cgcctacttc ctgttcctgc ggcgggacct gcaggctcag     720

tctagagctt ttcgtgccta tgtatctcga gtgtgtctcc gggaccagca ctactactcc     780

tatgtggagt tgcctctggc ctgcgaaggt ggccgctacg ggctgatcca ggctgcagct     840

gtggccacgt ccagggaggt ggcgcatggg gaggtgctct ttgcagcttt ctcctcggct     900

gcacccccca ctgtgggccg gcccccatcg gcggctgctg gggcatctgg agcctctgcc       960

ctctgtgcct tccccctgga tgaggtggac cggcttgcta atcgcacgcg agatgcctgc     1020

tacacccggg agggtcgtgc tgaggatggg accgaggtgg cctacatcga gtatgatgtc    1080

aattctgact gtgcacagct gccagtggac accctggatg cttatccctg tggctcagac    1140

cacacgccca gccccatggc cagccgggtc ccgctggaag ccacaccaat tctggagtgg    1200

ccagggattc agctaacagc tgtggcagtc accatggaag atggacacac catcgctttc    1260

ctgggtgata gtcaagggca gctgcacagg gtctacttgg gcccagggag cgatggccac 1320

ccatactcca cacagagcat ccagcagggg tctgcagtga gcagagacct cacctttgat 1380

gggacctttg agcacctgta tgtcatgacc cagagcacac ttctgaaggt tcctgtggct 1440

tcctgtgctc agcacctgga ctgtgcatct tgccttgctc acagggaccc atactgtggg 1500

tggtgcgtgc tccttggcag gtgcagtcgc cgttctgagt gctcgagggg ccagggccca 1560

gagcagtggc tatggagctt ccagcctgag ctgggctgtc tgcaagtggc agccatgagt 1620

cctgccaaca tcagccgaga ggagacgagg gaggttttcc tatcagtgcc agacctgcca 1680

cccctgtggc caggggagtc atattcctgc cactttgggg aacatcagag tcctgccctg 1740

ctgactggtt ctggtgtgat gtgcccctcc ccagacccta gtgaggcccc agtgctgccg 1800

agaggagccg actacgtatc cgtgagcgtg gagctcagat ttggcgctgt tgtgatcgcc 1860

aaaacttccc tctctttcta tgactgtgtg gcggtcactg aactccgccc atctgcgcag 1920

tgccaggcct gtgtgagcag ccgctggggg tgtaactggt gtgtctggca gcacctgtgc 1980

acccacaagg cctcgtgtga tgctgggccc atggttgcaa gccatcagag cccgcttgtc 2040

tccccagacc ctcctgcaag aggtggaccc agcccctccc cacccacagc ccccaaagcc 2100

ctggccaccc ctgctcctga cacccttccc gtggagcctg gggctccctc cacagccaca 2160

gcttcggaca tctcacctgg ggctagtcct tccctgctca gcccctgggg gccatgggca 2220

ggttctggct ccatatcttc ccctggctcc acagggtcgc ctctccatga ggagccctcc 2280

cctcccagcc cccaaaatgg acctggaacc gctgtccctg cccccactga cttcagaccc 2340

tcagccacac ctgaggacct cttggcctcc ccgctgtcac cctcagaggt agcagcagtg 2400

ccccctgcag accctggccc cgaggctctt catcccacag tgcccctgga cctgccccct 2460

gccactgttc ctgccaccac tttcccaggg gccatgggct ccgtgaagcc cgccctggac 2520

tggctcacga gagaaggcgg cgagctgccc gaggcggacg agtggacggg gggtgacgca 2580

cccgccttct ccacttccac cctcctctca ggtgatggag actcagcaga gcttgagggc 2640

cctcccgccc ccctcatcct cccgtccagc ctcgactacc agtatgacac ccccgggctc 2700

tgggagctgg aagaggcgac cttgggggca agctcctgcc cctgtgtgga gagcgttcag 2760

ggctccacgt tgatgccggt ccatgtggag cgggaaatcc ggctgctagg caggaacctg 2820

caccttttcc aggatggccc aggagacaat gagtgtgtga tggagctgga gggcctcgag 2880

gtggtggttg aggcccgggt cgagtgtgag ccacctccag atacccagtg ccatgtcacc 2940

tgccagcagc accagctcag ctatgaggct ctgcagccgg agctccgtgt ggggctgttt 3000

ctgcgtcggg ccggccgtct gcgtgtggac agtgctgagg ggctgcatgt ggtactgtat 3060

gactgttccg tgggacatgg agactgcagc cgctgccaaa ctgccatgcc ccagtatggc 3120

tgtgtgtggt gtgaggggga gcgtccacgt tgtgtgaccc gggaggcctg tggtgaggct 3180

gaggctgtgg ccacccagtg cccagcgccc ctcatccact cggtggagcc actgactggg 3240

cctgtagacg gaggcacccg tgtcaccatc aggggctcca acctgggcca gcatgtgcag 3300

gatgtgctgg gcatggtcac ggtggctgga gtgccctgtg ctgtggatgc ccaggagtac 3360

gaggtctcca gcagcctcgt gtgcatcacc ggggccagtg gggaggaggt ggccggcgcc 3420

acagcggtgg aggtgccggg aagaggacgt ggtgtctcag aacacgactt tgcctaccag 3480

gatccgaagg tccattccat cttcccggcc cgcggcccca gagctggggg cacccgtctc 3540

accctgaatg gctccaagct cctgactggg cggctggagg acatccgagt ggtggttgga 3600

gaccagcctt gtcacttgct gccggagcag cagtcagaac aactgcggtg tgagaccagc 3660

ccacgcccca cgcctgccac gctccctgtg gctgtgtggt ttggggccac ggagcggagg 3720

cttcaacgcg gacagttcaa gtataccttg gaccccaaca tcacctctgc tggccccacc 3780

aagagcttcc tcagtggagg acgtgagata tgcgtccgtg gccagaatct ggacgtggta 3840

cagacgccaa gaatccgggt gaccgtggtc tcgagaatgc tgcagcccag ccaggggctt 3900

ggacggaggc gtcgcgtggt cccggagacg gcatgttccc ttggaccctc ctgcagtagc 3960

cagcaatttg aggagccgtg ccatgtcaac tcctcccagc tcatcacgtg ccgcacacct 4020

gccctcccag gcctgcctga ggacccctgg gtccgggtgg aatttatcct tgacaacctg 4080

gtctttgact ttgcaacact gaaccccaca cctttctcct atgaggccga ccccaccctg 4140

cagccactca accctgagga ccccaccatg ccattccggc acaagcctgg gagtgtgttc 4200

tccgtggagg gggagaacct ggaccttgca atgtccaagg aggaggtggt ggctatgata 4260

ggggatggcc cctgtgtggt gaagacgctg acgcggcacc acctgtactg cgagcccccc 4320

gtggagcagc ccctgccacg gcaccatgcc ctccgagagg cacctgactc tttgcctgag 4380

ttcacggtgc agatggggaa cttgcgcttc tccctgggtc acgtgcagta tgacggcgag 4440

agccctgggg cttttcctgt ggcagcccag gtgggcttgg gggtgggcac ctctcttctg 4500

gctctgggtg tcatcatcat tgtcctcatg tacaggagga agagcaagca ggccctgagg 4560

gactataaga aggttcagat ccagctggag aatctggaga gcagtgtgcg ggaccgctgc 4620

aagaaggaat tcacagacct catgactgag atgaccgatc tcaccagtga cctcctgggc 4680

agcggcatcc ccttcctcga ctacaaggtg tatgcggaga ggatcttctt ccctgggcac 4740

cgcgagtcgc ccttgcaccg ggacctgggt gtgcctgaga gcagacggcc cactgtggag 4800

caagggctgg ggcagctctc taacctgctc aacagcaagc tcttcctcac caagttcatc 4860

cacacgctgg agagccagcg caccttttca gctcgggacc gtgcctacgt ggcatctctg 4920

ctcaccgtgg cactgcatgg gaagcttgag tatttcactg acatcctccg cactctgctc 4980

agtgacctgg ttgcccagta tgtggccaag aaccccaagc tgatgctgcg caggacagag 5040

actgtggtgg agaagctgct caccaactgg atgtccatct gtctgtatac cttcgtgagg 5100

gactccgtag ggagcctctt gtacatgctc tttcgaggga ttaagcacca agtggataag 5160

gggccagtgg acagtgtgac aggcaaggcc aaatacacct tgaacgacaa ccgcctgctc 5220

agagaggatg tggagtaccg tcccctgacc ttgaatgcac tattggctgt ggggcctggg 5280

gcaggagagg cccagggcgt gcccgtgaag gtcctagact gtgacaccat ctcccaggca 5340

aaggagaaga tgctggacca gctttataaa ggagtgcctc tcacccagcg gccagaccct 5400

cgcacccttg atgttgagtg gcggtctggg gtggccgggc acctcattct ttctgacgag 5460

gatgtcactt ctgaggtcca gggtctgtgg aggcgcctga acacactgca gcattacaag 5520

gtcccagatg gagcaactgt ggccctcgtc ccctgcctca ccaagcatgt gctccgggaa 5580

aaccaggatt atgtccctgg agagcggacc ccaatgctgg aggatgtaga tgaggggggc 5640

atccggccct ggcacctggt gaagccaagt gatgagccgg agccgcccag gcctcggagg 5700

ggcagccttc ggggcgggga gcgtgagcgc gccaaggcca tccctgagat ctacctgacc 5760

cgcctgctgt ccatgaaggg caccctgcag aagttcgtgg atgacctgtt ccaggtgatt 5820

ctcagcacca gccgccccgt gccgctcgct gtgaagtact tctttgacct gctggatgag 5880

caggcccagc agcatggcat ctccgaccag gacaccatcc acatctggaa gaccaacagc 5940

ttgcctctga ggttctggat caatataata aaaaacccgc agtttgtgtt cgacgtgcaa 6000

acatctgata acatggatgc ggtgctcctt gtcattgcac agaccttcat ggacgcctgc 6060

accctggccg accacaagct gggccgggac tccccgatca acaaacttct gtatgcacgg 6120

gacattcccc ggtacaagcg gatggtggaa aggtactatg cagacatcag acagactgtc 6180

ccagccagcg accaagagat gaactctgtc ctggctgaac tgtcctggaa ctactccgga 6240

gacctcgggg cgcgagtggc cctgcatgaa ctctacaagt acatcaacaa gtactatgac 6300

cagatcatca ctgccctgga ggaggatggc acggcccaga agatgcagct gggctatcgg 6360

ctccagcaga ttgcagctgc tgtggaaaac aaggtcacag atctatag 6408

<210> 2
<211> 5517
<212> DNA
<213> Homo sapiens

<220>
<223> Plexin-B2, human, cDNA (coding sequence)

<400> 2
atggcactgc agctctgggc cctgaccctg ctgggcctgc tgggcgcagg tgccagcctg 60

aggccccgca agctggactt cttccgcagc gagaaagagc tgaaccacct ggctgtggat 120

gaggcctcag gcgtggtgta cctgggggcg gtgaatgccc tctaccagct ggatgcgaag 180

ctgcagctgg agcagcaggt ggccacgggc ccggccctgg acaacaagaa gtgcacgccg 240

cccatcgagg ccagccagtg ccatgaggct gagatgactg acaatgtcaa ccagctgctg 300

ctgctcgacc ctcccaggaa gcgcctggtg gagtgcggca gcctcttcaa gggcatctgc 360

gctctgcgcg ccctgagcaa catctccctc cgcctgttct acgaggacgg cagcggggag 420

aagtctttcg tggccagcaa tgatgagggc gtggccacag tggggctggt gagctccacg 480

ggtcctggtg gtgaccgcgt gctgtttgtg ggcaaaggca atgggccaca cgacaacggc 540

atcatcgtga gcactcggct gttggaccgg actgacagca gggaggcctt tgaagcctac 600

acggaccacg ccacctacaa ggccggctac ctgtccacca acacacagca gttcgtggcg 660

gccttcgagg acggccccta cgtcttcttt gtcttcaacc agcaggacaa gcacccggcc 720

cggaaccgca cgctgctggc acgcatgtgc agagaagacc ccaactacta ctcctacctg 780

gagatggacc tgcagtgccg ggaccccgac atccacgccg ctgcctttgg cacctgcctg 840

gccgcctccg tggctgcgcc tggctctggc agggtgctat atgctgtctt cagcagagac 900

agccggagca gtgggggccc cggtgcgggc ctctgcctgt tcccgctgga caaggtgcac 960

gccaagatgg aggccaaccg caacgcctgt tacacaggca cccgggaggc ccgtgacatc 1020

ttctacaagc ccttccacgg cgatatccag tgcggcggcc acgcgccggg ctccagcaag 1080

agcttcccat gtggctcgga gcacctgccc tacccgctgg gcagccgcga cgggctcaga 1140

ggcacagccg tgctgcagcg tggaggcctg aacctcacgg ccgtgacggt cgccgccgag 1200

aacaaccaca ctgttgcttt tctgggcacc tctgatggcc ggatcctcaa ggtgtacctc 1260

accccagatg gcacctcctc agagtacgac tctatccttg tggagataaa caagagagtc 1320

aagcgcgacc tggtactgtc tggagacctg ggcagcctgt acgccatgac ccaggacaag 1380

gtgttccggc tgccggtgca ggagtgcctg agctacccga cctgcaccca gtgccgcgac 1440

tcccaggacc cctactgcgg ctggtgcgtc gtcgagggac gatgcacccg gaaggccgag 1500

tgtccgcggg ccgaggaggc cagccactgg ctgtggagcc gaagcaagtc ctgcgtggcc 1560

gtcaccagcg cccagccaca gaacatgagc cggcgggccc agggggaggt gcagctgacc 1620

gtcagccccc tccctgccct gagcgaggag gacgagttgc tgtgcctttt tggggagtcg 1680

ccgccacacc ccgcccgcgt ggagggcgag gccgtcatct gcaactcccc aagcagcatc 1740

cccgtcacac cgccaggcca ggaccacgtg gccgtgacca tccagctcct ccttagacga 1800

ggcaacatct tcctcacgtc ctaccagtac cccttctacg actgccgcca ggccatgagc 1860

ctggaggaga acctgccgtg catctcctgc gtgagcaacc gctggacctg ccagtgggac 1920

ctgcgctacc acgagtgccg ggaggcttcg cccaaccctg aggacggcat cgtccgtgcc 1980

cacatggagg acagctgtcc ccagttcctg ggacccagcc ccctggtgat ccccatgaac 2040

cacgagacag atgtgaactt ccagggcaag aacctggaca ccgtgaaggg ttcctccctg 2100

cacgtgggca gtgacttgct caagttcatg gagccggtga ccatgcagga atctgggacc 2160
ttcgcctttc ggaccccaaa gctgtcccac gatgccaacg agacgctgcc cctgcacctc 2220
tacgtcaagt cttacggcaa gaatatcgac agcaagctcc atgtgaccct ctacaactgc 2280
tcctttggcc gcagcgactg cagcctgtgc cgggccgcta accccgacta caggtgtgcg 2340
tggtgcgggg gccagagcag gtgcgtgtat gaggccctgt gcaacaccac ctccgagtgc 2400
ccgccgcccg tcatcaccag gatccagcct gagacgggcc ccctgggtgg gggcatccgc 2460
atcaccatcc tggggtccaa tttgggcgtc caagcagggg acatccagag gatctctgtg 2520
gccggccgga actgctcctt tcagccggaa cgttactccg tgtccacccg gatcgtgtgt 2580
gtgatcgagg ctgcggagac gcctttcacg gggggtgtcg aggtggacgt cttcgggaaa 2640
ctgggccgtt cgcctcccaa tgtccagttc accttccaac agcccaagcc tctcagtgtg 2700
gagccgcagc agggaccgca ggcgggcggc accacactga ccatccacgg cacccacctg 2760
gacacgggct cccaggagga cgtgcgggtg accctcaacg gcgtcccgtg taaagtgacg 2820
aagtttgggg cgcagctcca gtgtgtcact ggcccccagg cgacacgggg ccagatgctt 2880
ctggaggtct cctacggggg gtcccccgtg cccaaccccg gcatcttctt cacctaccgc 2940
gaaaaccccg tactgcgagc cttcgagccg ctacgaagct ttgccagtgg tggccgcagc 3000
atcaacgtca cgggtcaggg cttcagcctg atccagaggt ttgccatggt ggtcatcgcg 3060
gagcccctgc agtcctggca gccgccgcgg gaggctgaat ccctgcagcc catgacggtg 3120
gtgggtacag actacgtgtt ccacaatgac accaaggtcg tcttcctgtc cccggctgtg 3180
cctgaggagc cagaggccta caacctcacg gtgctgatcg agatggacgg gcaccgtgcc 3240
ctgctcagaa cagaggccgg ggccttcgag tacgtgcctg accccacctt tgagaacttc 3300
acaggtggcg tcaagaagca ggtcaacaag ctcatccacg cccggggcac caatctgaac 3360
aaggcgatga cgctgcagga ggccgaggcc ttcgtgggtg ccgagcgctg caccatgaag 3420
acgctgacgg agaccgacct gtactgtgag cccccggagg tgcagccccc gcccaagcgg 3480
cggcagaaac gagacaccac acacaacctg cccgagttca ttgtgaagtt cggctctcgc 3540
gagtgggtgc tgggccgcgt ggagtacgac acacgggtga gcgacgtgcc gctcagcctc 3600
atcttgccgc tggtcatcgt gcccatggtg gtcgtcatcg cggtgtctgt ctactgctac 3660
tggaggaaga gccagcaggc cgaacgagag tatgagaaga tcaagtccca gctggagggc 3720
ctggaggaga gcgtgcggga ccgctgcaag aaggaattca cagacctgat gatcgagatg 3780
gaggaccaga ccaacgacgt gcacgaggcc ggcatccccg tgctggacta caagacctac 3840
accgaccgcg tcttcttcct gccctccaag gacggcgaca aggacgtgat gatcaccggc 3900
aagctggaca tccctgagcc gcggcggccg gtggtggagc aggccctcta ccagttctcc 3960

aacctgctga acagcaagtc tttcctcatc aatttcatcc acaccctgga gaaccagcgg 4020

gagttctcgg cccgcgccaa ggtctacttc gcgtccctgc tgacggtggc gctgcacggg 4080

aaactggagt actacacgga catcatgcac acgctcttcc tggagctcct ggagcagtac 4140

gtggtggcca agaaccccaa gctgatgctg cgcaggtctg agactgtggt ggagaggatg 4200

ctgtccaact ggatgtccat ctgcctgtac cagtacctca aggacagtgc cggggagccc 4260

ctgtacaagc tcttcaaggc catcaaacat caggtggaaa agggcccggt ggatgcggta 4320

cagaagaagg ccaagtacac tctcaacgac acggggctgc tgggggatga tgtggagtac 4380

gcacccctga cggtgagcgt gatcgtgcag gacgagggag tggacgccat cccggtgaag 4440

gtcctcaact gtgacaccat ctcccaggtc aaggagaaga tcattgacca ggtgtaccgt 4500

gggcagccct gctcctgctg gcccaggcca gacagcgtgg tcctggagtg gcgtccgggc 4560

tccacagcgc agatcctgtc ggacctggac ctgacgtcac agcgggaggg ccggtggaag 4620

cgcgtcaaca cccttatgca ctacaatgtc cgggatggag ccaccctcat cctgtccaag 4680

gtgggggtct cccagcagcc ggaggacagc cagcaggacc tgcctgggga gcgccatgcc 4740

ctcctggagg aggagaaccg ggtgtggcac ctggtgcggc cgaccgacga ggtggacgag 4800

ggcaagtcca agagaggcag cgtgaaagag aaggagcgga cgaaggccat caccgagatc 4860

tacctgacgc ggctgctctc agtcaagggc acactgcagc agtttgtgga caacttcttc 4920

cagagcgtgc tggcgcctgg gcacgcggtg ccacctgcag tcaagtactt cttcgacttc 4980

ctggacgagc aggcagagaa gcacaacatc caggatgaag acaccatcca catctggaag 5040

acgaacagct taccgctccg gttctgggtg aacatcctca agaaccccca cttcatcttt 5100

gacgtgcatg tccacgaggt ggtggacgcc tcgctgtcag tcatcgcgca gaccttcatg 5160

gatgcctgca cgcgcacgga gcataagctg agccgcgatt ctcccagcaa caagctgctg 5220

tacgccaagg agatctccac ctacaagaag atggtggagg attactacaa ggggatccgg 5280

cagatggtgc aggtcagcga ccaggacatg aacacacacc tggcagagat ttcccgggcg 5340

cacacggact ccttgaacac cctcgtggca ctccaccagc tctaccaata cacgcagaag 5400

tactatgacg agatcatcaa tgccttggag gaggatcctg ccgcccagaa gatgcagctg 5460

gccttccgcc tgcagcagat tgccgctgca ctggagaaca aggtcactga cctctga 5517

<210> 3
<211> 2135
<212> PRT
<213> Homo sapiens

<220>
<223> Plexin-B1, human, Protein

<400> 3
Met Pro Ala Leu Gly Pro Ala Leu Leu Gln Ala Leu Trp Ala Gly Trp

1               5                   10                  15
Val Leu Thr Leu Gln Pro Leu Pro Pro Thr Ala Phe Thr Pro Asn Gly
            20                  25                  30
Thr Tyr Leu Gln His Leu Ala Arg Asp Pro Thr Ser Gly Thr Leu Tyr
        35                  40                  45
Leu Gly Ala Thr Asn Phe Leu Phe Gln Leu Ser Pro Gly Leu Gln Leu
    50                  55                  60
Glu Ala Thr Val Ser Thr Gly Pro Val Leu Asp Ser Arg Asp Cys Leu
65                  70                  75                  80
Pro Pro Val Met Pro Asp Glu Cys Pro Gln Ala Gln Pro Thr Asn Asn
                85                  90                  95
Pro Asn Gln Leu Leu Leu Val Ser Pro Gly Ala Leu Val Val Cys Gly
            100                 105                 110
Ser Val His Gln Gly Val Cys Glu Gln Arg Arg Leu Gly Gln Leu Glu
        115                 120                 125
Gln Leu Leu Leu Arg Pro Glu Arg Pro Gly Asp Thr Gln Tyr Val Ala
    130                 135                 140
Ala Asn Asp Pro Ala Val Ser Thr Val Gly Leu Val Ala Gln Gly Leu
145                 150                 155                 160
Ala Gly Glu Pro Leu Leu Phe Val Gly Arg Gly Tyr Thr Ser Arg Gly
                165                 170                 175
Val Gly Gly Gly Ile Pro Pro Ile Thr Thr Arg Ala Leu Trp Pro Pro
            180                 185                 190
Asp Pro Gln Ala Ala Phe Ser Tyr Glu Glu Thr Ala Lys Leu Ala Val
        195                 200                 205
Gly Arg Leu Ser Glu Tyr Ser His His Phe Val Ser Ala Phe Ala Arg
    210                 215                 220
Gly Ala Ser Ala Tyr Phe Leu Phe Leu Arg Arg Asp Leu Gln Ala Gln
225                 230                 235                 240
Ser Arg Ala Phe Arg Ala Tyr Val Ser Arg Val Cys Leu Arg Asp Gln
                245                 250                 255
His Tyr Tyr Ser Tyr Val Glu Leu Pro Leu Ala Cys Glu Gly Gly Arg
            260                 265                 270
Tyr Gly Leu Ile Gln Ala Ala Ala Val Ala Thr Ser Arg Glu Val Ala
        275                 280                 285
His Gly Glu Val Leu Phe Ala Ala Phe Ser Ser Ala Ala Pro Pro Thr
    290                 295                 300
Val Gly Arg Pro Pro Ser Ala Ala Ala Gly Ala Ser Gly Ala Ser Ala
305                 310                 315                 320
Leu Cys Ala Phe Pro Leu Asp Glu Val Asp Arg Leu Ala Asn Arg Thr
                325                 330                 335
Arg Asp Ala Cys Tyr Thr Arg Glu Gly Arg Ala Glu Asp Gly Thr Glu
            340                 345                 350
Val Ala Tyr Ile Glu Tyr Asp Val Asn Ser Asp Cys Ala Gln Leu Pro
        355                 360                 365
Val Asp Thr Leu Asp Ala Tyr Pro Cys Gly Ser Asp His Thr Pro Ser
    370                 375                 380
Pro Met Ala Ser Arg Val Pro Leu Glu Ala Thr Pro Ile Leu Glu Trp
385                 390                 395                 400
Pro Gly Ile Gln Leu Thr Ala Val Ala Val Thr Met Glu Asp Gly His
                405                 410                 415
Thr Ile Ala Phe Leu Gly Asp Ser Gln Gly Gln Leu His Arg Val Tyr
            420                 425                 430
Leu Gly Pro Gly Ser Asp Gly His Pro Tyr Ser Thr Gln Ser Ile Gln
        435                 440                 445
Gln Gly Ser Ala Val Ser Arg Asp Leu Thr Phe Asp Gly Thr Phe Glu
    450                 455                 460
His Leu Tyr Val Met Thr Gln Ser Thr Leu Leu Lys Val Pro Val Ala
465                 470                 475                 480
Ser Cys Ala Gln His Leu Asp Cys Ala Ser Cys Leu Ala His Arg Asp
                485                 490                 495
Pro Tyr Cys Gly Trp Cys Val Leu Leu Gly Arg Cys Ser Arg Arg Ser
            500                 505                 510

Glu Cys Ser Arg Gly Gln Gly Pro Glu Gln Trp Leu Trp Ser Phe Gln
        515                 520                 525
Pro Glu Leu Gly Cys Leu Gln Val Ala Ala Met Ser Pro Ala Asn Ile
    530                 535                 540
Ser Arg Glu Glu Thr Arg Glu Val Phe Leu Ser Val Pro Asp Leu Pro
545                 550                 555                 560
Pro Leu Trp Pro Gly Glu Ser Tyr Ser Cys His Phe Gly Glu His Gln
                565                 570                 575
Ser Pro Ala Leu Leu Thr Gly Ser Gly Val Met Cys Pro Ser Pro Asp
            580                 585                 590
Pro Ser Glu Ala Pro Val Leu Pro Arg Gly Ala Asp Tyr Val Ser Val
        595                 600                 605
Ser Val Glu Leu Arg Phe Gly Ala Val Val Ile Ala Lys Thr Ser Leu
    610                 615                 620
Ser Phe Tyr Asp Cys Val Ala Val Thr Glu Leu Arg Pro Ser Ala Gln
625                 630                 635                 640
Cys Gln Ala Cys Val Ser Ser Arg Trp Gly Cys Asn Trp Cys Val Trp
                645                 650                 655
Gln His Leu Cys Thr His Lys Ala Ser Cys Asp Ala Gly Pro Met Val
            660                 665                 670
Ala Ser His Gln Ser Pro Leu Val Ser Pro Asp Pro Pro Ala Arg Gly
        675                 680                 685
Gly Pro Ser Pro Ser Pro Pro Thr Ala Pro Lys Ala Leu Ala Thr Pro
    690                 695                 700
Ala Pro Asp Thr Leu Pro Val Glu Pro Gly Ala Pro Ser Thr Ala Thr
705                 710                 715                 720
Ala Ser Asp Ile Ser Pro Gly Ala Ser Pro Ser Leu Leu Ser Pro Trp
                725                 730                 735
Gly Pro Trp Ala Gly Ser Gly Ser Ile Ser Ser Pro Gly Ser Thr Gly
            740                 745                 750
Ser Pro Leu His Glu Glu Pro Ser Pro Pro Ser Pro Gln Asn Gly Pro
        755                 760                 765
Gly Thr Ala Val Pro Ala Pro Thr Asp Phe Arg Pro Ser Ala Thr Pro
    770                 775                 780
Glu Asp Leu Leu Ala Ser Pro Leu Ser Pro Ser Glu Val Ala Ala Val
785                 790                 795                 800
Pro Pro Ala Asp Pro Gly Pro Glu Ala Leu His Pro Thr Val Pro Leu
                805                 810                 815
Asp Leu Pro Pro Ala Thr Val Pro Ala Thr Thr Phe Pro Gly Ala Met
            820                 825                 830
Gly Ser Val Lys Pro Ala Leu Asp Trp Leu Thr Arg Glu Gly Gly Glu
        835                 840                 845
Leu Pro Glu Ala Asp Glu Trp Thr Gly Gly Asp Ala Pro Ala Phe Ser
    850                 855                 860
Thr Ser Thr Leu Leu Ser Gly Asp Gly Asp Ser Ala Glu Leu Glu Gly
865                 870                 875                 880
Pro Pro Ala Pro Leu Ile Leu Pro Ser Ser Leu Asp Tyr Gln Tyr Asp
                885                 890                 895
Thr Pro Gly Leu Trp Glu Leu Glu Glu Ala Thr Leu Gly Ala Ser Ser
            900                 905                 910
Cys Pro Cys Val Glu Ser Val Gln Gly Ser Thr Leu Met Pro Val His
        915                 920                 925
Val Glu Arg Glu Ile Arg Leu Leu Gly Arg Asn Leu His Leu Phe Gln
    930                 935                 940
Asp Gly Pro Gly Asp Asn Glu Cys Val Met Glu Leu Glu Gly Leu Glu
945                 950                 955                 960
Val Val Val Glu Ala Arg Val Glu Cys Glu Pro Pro Pro Asp Thr Gln
                965                 970                 975
Cys His Val Thr Cys Gln Gln His Gln Leu Ser Tyr Glu Ala Leu Gln
            980                 985                 990
Pro Glu Leu Arg Val Gly Leu Phe Leu Arg Arg Ala Gly Arg Leu Arg
        995                 1000                1005
Val Asp Ser Ala Glu Gly Leu His Val Val Leu Tyr Asp Cys Ser Val

1010                    1015                    1020
Gly His Gly Asp Cys Ser Arg Cys Gln Thr Ala Met Pro Gln Tyr Gly
1025                    1030                    1035                    1040
Cys Val Trp Cys Glu Gly Glu Arg Pro Arg Cys Val Thr Arg Glu Ala
                1045                    1050                    1055
Cys Gly Glu Ala Glu Ala Val Ala Thr Gln Cys Pro Ala Pro Leu Ile
            1060                    1065                    1070
His Ser Val Glu Pro Leu Thr Gly Pro Val Asp Gly Gly Thr Arg Val
        1075                    1080                    1085
Thr Ile Arg Gly Ser Asn Leu Gly Gln His Val Gln Asp Val Leu Gly
    1090                    1095                    1100
Met Val Thr Val Ala Gly Val Pro Cys Ala Val Asp Ala Gln Glu Tyr
1105                    1110                    1115                    1120
Glu Val Ser Ser Ser Leu Val Cys Ile Thr Gly Ala Ser Gly Glu Glu
                1125                    1130                    1135
Val Ala Gly Ala Thr Ala Val Glu Val Pro Gly Arg Gly Arg Gly Val
            1140                    1145                    1150
Ser Glu His Asp Phe Ala Tyr Gln Asp Pro Lys Val His Ser Ile Phe
        1155                    1160                    1165
Pro Ala Arg Gly Pro Arg Ala Gly Gly Thr Arg Leu Thr Leu Asn Gly
    1170                    1175                    1180
Ser Lys Leu Leu Thr Gly Arg Leu Glu Asp Ile Arg Val Val Val Gly
1185                    1190                    1195                    1200
Asp Gln Pro Cys His Leu Leu Pro Glu Gln Gln Ser Glu Gln Leu Arg
                1205                    1210                    1215
Cys Glu Thr Ser Pro Arg Pro Thr Pro Ala Thr Leu Pro Val Ala Val
            1220                    1225                    1230
Trp Phe Gly Ala Thr Glu Arg Arg Leu Gln Arg Gly Gln Phe Lys Tyr
        1235                    1240                    1245
Thr Leu Asp Pro Asn Ile Thr Ser Ala Gly Pro Thr Lys Ser Phe Leu
    1250                    1255                    1260
Ser Gly Gly Arg Glu Ile Cys Val Arg Gly Gln Asn Leu Asp Val Val
1265                    1270                    1275                    1280
Gln Thr Pro Arg Ile Arg Val Thr Val Val Ser Arg Met Leu Gln Pro
                1285                    1290                    1295
Ser Gln Gly Leu Gly Arg Arg Arg Arg Val Val Pro Glu Thr Ala Cys
            1300                    1305                    1310
Ser Leu Gly Pro Ser Cys Ser Ser Gln Gln Phe Glu Glu Pro Cys His
        1315                    1320                    1325
Val Asn Ser Ser Gln Leu Ile Thr Cys Arg Thr Pro Ala Leu Pro Gly
    1330                    1335                    1340
Leu Pro Glu Asp Pro Trp Val Arg Val Glu Phe Ile Leu Asp Asn Leu
1345                    1350                    1355                    1360
Val Phe Asp Phe Ala Thr Leu Asn Pro Thr Pro Phe Ser Tyr Glu Ala
                1365                    1370                    1375
Asp Pro Thr Leu Gln Pro Leu Asn Pro Glu Asp Pro Thr Met Pro Phe
            1380                    1385                    1390
Arg His Lys Pro Gly Ser Val Phe Ser Val Glu Gly Glu Asn Leu Asp
        1395                    1400                    1405
Leu Ala Met Ser Lys Glu Glu Val Val Ala Met Ile Gly Asp Gly Pro
    1410                    1415                    1420
Cys Val Val Lys Thr Leu Thr Arg His His Leu Tyr Cys Glu Pro Pro
1425                    1430                    1435                    1440
Val Glu Gln Pro Leu Pro Arg His His Ala Leu Arg Glu Ala Pro Asp
                1445                    1450                    1455
Ser Leu Pro Glu Phe Thr Val Gln Met Gly Asn Leu Arg Phe Ser Leu
            1460                    1465                    1470
Gly His Val Gln Tyr Asp Gly Glu Ser Pro Gly Ala Phe Pro Val Ala
        1475                    1480                    1485
Ala Gln Val Gly Leu Gly Val Gly Thr Ser Leu Leu Ala Leu Gly Val
    1490                    1495                    1500
Ile Ile Ile Val Leu Met Tyr Arg Arg Lys Ser Lys Gln Ala Leu Arg
1505                    1510                    1515                    1520

Asp Tyr Lys Lys Val Gln Ile Gln Leu Glu Asn Leu Glu Ser Ser Val
                1525                1530                1535
Arg Asp Arg Cys Lys Lys Glu Phe Thr Asp Leu Met Thr Glu Met Thr
            1540                1545                1550
Asp Leu Thr Ser Asp Leu Leu Gly Ser Gly Ile Pro Phe Leu Asp Tyr
        1555                1560                1565
Lys Val Tyr Ala Glu Arg Ile Phe Phe Pro Gly His Arg Glu Ser Pro
    1570                1575                1580
Leu His Arg Asp Leu Gly Val Pro Glu Ser Arg Arg Pro Thr Val Glu
1585                1590                1595                1600
Gln Gly Leu Gly Gln Leu Ser Asn Leu Leu Asn Ser Lys Leu Phe Leu
                1605                1610                1615
Thr Lys Phe Ile His Thr Leu Glu Ser Gln Arg Thr Phe Ser Ala Arg
            1620                1625                1630
Asp Arg Ala Tyr Val Ala Ser Leu Leu Thr Val Ala Leu His Gly Lys
        1635                1640                1645
Leu Glu Tyr Phe Thr Asp Ile Leu Arg Thr Leu Leu Ser Asp Leu Val
    1650                1655                1660
Ala Gln Tyr Val Ala Lys Asn Pro Lys Leu Met Leu Arg Arg Thr Glu
1665                1670                1675                1680
Thr Val Val Glu Lys Leu Leu Thr Asn Trp Met Ser Ile Cys Leu Tyr
                1685                1690                1695
Thr Phe Val Arg Asp Ser Val Gly Glu Pro Leu Tyr Met Leu Phe Arg
            1700                1705                1710
Gly Ile Lys His Gln Val Asp Lys Gly Pro Val Asp Ser Val Thr Gly
        1715                1720                1725
Lys Ala Lys Tyr Thr Leu Asn Asp Asn Arg Leu Leu Arg Glu Asp Val
    1730                1735                1740
Glu Tyr Arg Pro Leu Thr Leu Asn Ala Leu Leu Ala Val Gly Pro Gly
1745                1750                1755                1760
Ala Gly Glu Ala Gln Gly Val Pro Val Lys Val Leu Asp Cys Asp Thr
                1765                1770                1775
Ile Ser Gln Ala Lys Glu Lys Met Leu Asp Gln Leu Tyr Lys Gly Val
            1780                1785                1790
Pro Leu Thr Gln Arg Pro Asp Pro Arg Thr Leu Asp Val Glu Trp Arg
        1795                1800                1805
Ser Gly Val Ala Gly His Leu Ile Leu Ser Asp Glu Asp Val Thr Ser
    1810                1815                1820
Glu Val Gln Gly Leu Trp Arg Arg Leu Asn Thr Leu Gln His Tyr Lys
1825                1830                1835                1840
Val Pro Asp Gly Ala Thr Val Ala Leu Val Pro Cys Leu Thr Lys His
                1845                1850                1855
Val Leu Arg Glu Asn Gln Asp Tyr Val Pro Gly Glu Arg Thr Pro Met
            1860                1865                1870
Leu Glu Asp Val Asp Glu Gly Gly Ile Arg Pro Trp His Leu Val Lys
        1875                1880                1885
Pro Ser Asp Glu Pro Glu Pro Pro Arg Pro Arg Arg Gly Ser Leu Arg
    1890                1895                1900
Gly Gly Glu Arg Glu Arg Ala Lys Ala Ile Pro Glu Ile Tyr Leu Thr
1905                1910                1915                1920
Arg Leu Leu Ser Met Lys Gly Thr Leu Gln Lys Phe Val Asp Asp Leu
                1925                1930                1935
Phe Gln Val Ile Leu Ser Thr Ser Arg Pro Val Pro Leu Ala Val Lys
            1940                1945                1950
Tyr Phe Phe Asp Leu Leu Asp Glu Gln Ala Gln Gln His Gly Ile Ser
        1955                1960                1965
Asp Gln Asp Thr Ile His Ile Trp Lys Thr Asn Ser Leu Pro Leu Arg
    1970                1975                1980
Phe Trp Ile Asn Ile Ile Lys Asn Pro Gln Phe Val Phe Asp Val Gln
1985                1990                1995                2000
Thr Ser Asp Asn Met Asp Ala Val Leu Leu Val Ile Ala Gln Thr Phe
                2005                2010                2015
Met Asp Ala Cys Thr Leu Ala Asp His Lys Leu Gly Arg Asp Ser Pro

2020                    2025                    2030
Ile Asn Lys Leu Leu Tyr Ala Arg Asp Ile Pro Arg Tyr Lys Arg Met
        2035                    2040                    2045
Val Glu Arg Tyr Tyr Ala Asp Ile Arg Gln Thr Val Pro Ala Ser Asp
    2050                    2055                    2060
Gln Glu Met Asn Ser Val Leu Ala Glu Leu Ser Trp Asn Tyr Ser Gly
2065                    2070                    2075                    2080
Asp Leu Gly Ala Arg Val Ala Leu His Glu Leu Tyr Lys Tyr Ile Asn
                2085                    2090                    2095
Lys Tyr Tyr Asp Gln Ile Ile Thr Ala Leu Glu Glu Asp Gly Thr Ala
            2100                    2105                    2110
Gln Lys Met Gln Leu Gly Tyr Arg Leu Gln Gln Ile Ala Ala Ala Val
        2115                    2120                    2125
Glu Asn Lys Val Thr Asp Leu
    2130                    2135

<210> 4
<211> 1838
<212> PRT
<213> Homo sapiens

<220>
<223> Plexin-B2, human, Protein

<400> 4
Met Ala Leu Gln Leu Trp Ala Leu Thr Leu Leu Gly Leu Leu Gly Ala
1               5                   10                  15
Gly Ala Ser Leu Arg Pro Arg Lys Leu Asp Phe Phe Arg Ser Glu Lys
            20                  25                  30
Glu Leu Asn His Leu Ala Val Asp Glu Ala Ser Gly Val Val Tyr Leu
        35                  40                  45
Gly Ala Val Asn Ala Leu Tyr Gln Leu Asp Ala Lys Leu Gln Leu Glu
    50                  55                  60
Gln Gln Val Ala Thr Gly Pro Ala Leu Asp Asn Lys Lys Cys Thr Pro
65                  70                  75                  80
Pro Ile Glu Ala Ser Gln Cys His Glu Ala Glu Met Thr Asp Asn Val
                85                  90                  95
Asn Gln Leu Leu Leu Leu Asp Pro Pro Arg Lys Arg Leu Val Glu Cys
            100                 105                 110
Gly Ser Leu Phe Lys Gly Ile Cys Ala Leu Arg Ala Leu Ser Asn Ile
        115                 120                 125
Ser Leu Arg Leu Phe Tyr Glu Asp Gly Ser Gly Glu Lys Ser Phe Val
    130                 135                 140
Ala Ser Asn Asp Glu Gly Val Ala Thr Val Gly Leu Val Ser Ser Thr
145                 150                 155                 160
Gly Pro Gly Gly Asp Arg Val Leu Phe Val Gly Lys Gly Asn Gly Pro
                165                 170                 175
His Asp Asn Gly Ile Ile Val Ser Thr Arg Leu Leu Asp Arg Thr Asp
            180                 185                 190
Ser Arg Glu Ala Phe Glu Ala Tyr Thr Asp His Ala Thr Tyr Lys Ala
        195                 200                 205
Gly Tyr Leu Ser Thr Asn Thr Gln Gln Phe Val Ala Ala Phe Glu Asp
    210                 215                 220
Gly Pro Tyr Val Phe Phe Val Phe Asn Gln Gln Asp Lys His Pro Ala
225                 230                 235                 240
Arg Asn Arg Thr Leu Leu Ala Arg Met Cys Arg Glu Asp Pro Asn Tyr
                245                 250                 255
Tyr Ser Tyr Leu Glu Met Asp Leu Gln Cys Arg Asp Pro Asp Ile His
            260                 265                 270
Ala Ala Ala Phe Gly Thr Cys Leu Ala Ala Ser Val Ala Ala Pro Gly
        275                 280                 285
Ser Gly Arg Val Leu Tyr Ala Val Phe Ser Arg Asp Ser Arg Ser Ser
    290                 295                 300

Gly Gly Pro Gly Ala Gly Leu Cys Leu Phe Pro Leu Asp Lys Val His
305                 310                 315                 320
Ala Lys Met Glu Ala Asn Arg Asn Ala Cys Tyr Thr Gly Thr Arg Glu
                325                 330                 335
Ala Arg Asp Ile Phe Tyr Lys Pro Phe His Gly Asp Ile Gln Cys Gly
            340                 345                 350
Gly His Ala Pro Gly Ser Ser Lys Ser Phe Pro Cys Gly Ser Glu His
        355                 360                 365
Leu Pro Tyr Pro Leu Gly Ser Arg Asp Gly Leu Arg Gly Thr Ala Val
    370                 375                 380
Leu Gln Arg Gly Gly Leu Asn Leu Thr Ala Val Thr Val Ala Ala Glu
385                 390                 395                 400
Asn Asn His Thr Val Ala Phe Leu Gly Thr Ser Asp Gly Arg Ile Leu
                405                 410                 415
Lys Val Tyr Leu Thr Pro Asp Gly Thr Ser Ser Glu Tyr Asp Ser Ile
            420                 425                 430
Leu Val Glu Ile Asn Lys Arg Val Lys Arg Asp Leu Val Leu Ser Gly
        435                 440                 445
Asp Leu Gly Ser Leu Tyr Ala Met Thr Gln Asp Lys Val Phe Arg Leu
    450                 455                 460
Pro Val Gln Glu Cys Leu Ser Tyr Pro Thr Cys Thr Gln Cys Arg Asp
465                 470                 475                 480
Ser Gln Asp Pro Tyr Cys Gly Trp Cys Val Val Glu Gly Arg Cys Thr
                485                 490                 495
Arg Lys Ala Glu Cys Pro Arg Ala Glu Glu Ala Ser His Trp Leu Trp
            500                 505                 510
Ser Arg Ser Lys Ser Cys Val Ala Val Thr Ser Ala Gln Pro Gln Asn
        515                 520                 525
Met Ser Arg Arg Ala Gln Gly Glu Val Gln Leu Thr Val Ser Pro Leu
    530                 535                 540
Pro Ala Leu Ser Glu Glu Asp Glu Leu Leu Cys Leu Phe Gly Glu Ser
545                 550                 555                 560
Pro Pro His Pro Ala Arg Val Glu Gly Glu Ala Val Ile Cys Asn Ser
                565                 570                 575
Pro Ser Ser Ile Pro Val Thr Pro Pro Gly Gln Asp His Val Ala Val
            580                 585                 590
Thr Ile Gln Leu Leu Leu Arg Arg Gly Asn Ile Phe Leu Thr Ser Tyr
        595                 600                 605
Gln Tyr Pro Phe Tyr Asp Cys Arg Gln Ala Met Ser Leu Glu Glu Asn
    610                 615                 620
Leu Pro Cys Ile Ser Cys Val Ser Asn Arg Trp Thr Cys Gln Trp Asp
625                 630                 635                 640
Leu Arg Tyr His Glu Cys Arg Glu Ala Ser Pro Asn Pro Glu Asp Gly
                645                 650                 655
Ile Val Arg Ala His Met Glu Asp Ser Cys Pro Gln Phe Leu Gly Pro
            660                 665                 670
Ser Pro Leu Val Ile Pro Met Asn His Glu Thr Asp Val Asn Phe Gln
        675                 680                 685
Gly Lys Asn Leu Asp Thr Val Lys Gly Ser Ser Leu His Val Gly Ser
    690                 695                 700
Asp Leu Leu Lys Phe Met Glu Pro Val Thr Met Gln Glu Ser Gly Thr
705                 710                 715                 720
Phe Ala Phe Arg Thr Pro Lys Leu Ser His Asp Ala Asn Glu Thr Leu
                725                 730                 735
Pro Leu His Leu Tyr Val Lys Ser Tyr Gly Lys Asn Ile Asp Ser Lys
            740                 745                 750
Leu His Val Thr Leu Tyr Asn Cys Ser Phe Gly Arg Ser Asp Cys Ser
        755                 760                 765
Leu Cys Arg Ala Ala Asn Pro Asp Tyr Arg Cys Ala Trp Cys Gly Gly
    770                 775                 780
Gln Ser Arg Cys Val Tyr Glu Ala Leu Cys Asn Thr Thr Ser Glu Cys
785                 790                 795                 800
Pro Pro Pro Val Ile Thr Arg Ile Gln Pro Glu Thr Gly Pro Leu Gly

                805                 810                 815
Gly Gly Ile Arg Ile Thr Ile Leu Gly Ser Asn Leu Gly Val Gln Ala
            820                 825                 830
Gly Asp Ile Gln Arg Ile Ser Val Ala Gly Arg Asn Cys Ser Phe Gln
        835                 840                 845
Pro Glu Arg Tyr Ser Val Ser Thr Arg Ile Val Cys Val Ile Glu Ala
    850                 855                 860
Ala Glu Thr Pro Phe Thr Gly Gly Val Glu Val Asp Val Phe Gly Lys
865                 870                 875                 880
Leu Gly Arg Ser Pro Pro Asn Val Gln Phe Thr Phe Gln Gln Pro Lys
                885                 890                 895
Pro Leu Ser Val Glu Pro Gln Gln Gly Pro Gln Ala Gly Gly Thr Thr
            900                 905                 910
Leu Thr Ile His Gly Thr His Leu Asp Thr Gly Ser Gln Glu Asp Val
        915                 920                 925
Arg Val Thr Leu Asn Gly Val Pro Cys Lys Val Thr Lys Phe Gly Ala
    930                 935                 940
Gln Leu Gln Cys Val Thr Gly Pro Gln Ala Thr Arg Gly Gln Met Leu
945                 950                 955                 960
Leu Glu Val Ser Tyr Gly Gly Ser Pro Val Pro Asn Pro Gly Ile Phe
                965                 970                 975
Phe Thr Tyr Arg Glu Asn Pro Val Leu Arg Ala Phe Glu Pro Leu Arg
            980                 985                 990
Ser Phe Ala Ser Gly Gly Arg Ser Ile Asn Val Thr Gly Gln Gly Phe
        995                 1000                1005
Ser Leu Ile Gln Arg Phe Ala Met Val Val Ile Ala Glu Pro Leu Gln
    1010                1015                1020
Ser Trp Gln Pro Pro Arg Glu Ala Glu Ser Leu Gln Pro Met Thr Val
1025                1030                1035                1040
Val Gly Thr Asp Tyr Val Phe His Asn Asp Thr Lys Val Val Phe Leu
                1045                1050                1055
Ser Pro Ala Val Pro Glu Glu Pro Glu Ala Tyr Asn Leu Thr Val Leu
            1060                1065                1070
Ile Glu Met Asp Gly His Arg Ala Leu Leu Arg Thr Glu Ala Gly Ala
        1075                1080                1085
Phe Glu Tyr Val Pro Asp Pro Thr Phe Glu Asn Phe Thr Gly Gly Val
    1090                1095                1100
Lys Lys Gln Val Asn Lys Leu Ile His Ala Arg Gly Thr Asn Leu Asn
1105                1110                1115                1120
Lys Ala Met Thr Leu Gln Glu Ala Glu Ala Phe Val Gly Ala Glu Arg
                1125                1130                1135
Cys Thr Met Lys Thr Leu Thr Glu Thr Asp Leu Tyr Cys Glu Pro Pro
            1140                1145                1150
Glu Val Gln Pro Pro Pro Lys Arg Arg Gln Lys Arg Asp Thr Thr His
        1155                1160                1165
Asn Leu Pro Glu Phe Ile Val Lys Phe Gly Ser Arg Glu Trp Val Leu
    1170                1175                1180
Gly Arg Val Glu Tyr Asp Thr Arg Val Ser Asp Val Pro Leu Ser Leu
1185                1190                1195                1200
Ile Leu Pro Leu Val Ile Val Pro Met Val Val Val Ile Ala Val Ser
                1205                1210                1215
Val Tyr Cys Tyr Trp Arg Lys Ser Gln Gln Ala Glu Arg Glu Tyr Glu
            1220                1225                1230
Lys Ile Lys Ser Gln Leu Glu Gly Leu Glu Glu Ser Val Arg Asp Arg
        1235                1240                1245
Cys Lys Lys Glu Phe Thr Asp Leu Met Ile Glu Met Glu Asp Gln Thr
    1250                1255                1260
Asn Asp Val His Glu Ala Gly Ile Pro Val Leu Asp Tyr Lys Thr Tyr
1265                1270                1275                1280
Thr Asp Arg Val Phe Phe Leu Pro Ser Lys Asp Gly Asp Lys Asp Val
                1285                1290                1295
Met Ile Thr Gly Lys Leu Asp Ile Pro Glu Pro Arg Arg Pro Val Val
            1300                1305                1310

Glu Gln Ala Leu Tyr Gln Phe Ser Asn Leu Leu Asn Ser Lys Ser Phe
        1315                1320                1325
Leu Ile Asn Phe Ile His Thr Leu Glu Asn Gln Arg Glu Phe Ser Ala
    1330                1335                1340
Arg Ala Lys Val Tyr Phe Ala Ser Leu Leu Thr Val Ala Leu His Gly
1345                1350                1355                1360
Lys Leu Glu Tyr Tyr Thr Asp Ile Met His Thr Leu Phe Leu Glu Leu
                1365                1370                1375
Leu Glu Gln Tyr Val Val Ala Lys Asn Pro Lys Leu Met Leu Arg Arg
            1380                1385                1390
Ser Glu Thr Val Val Glu Arg Met Leu Ser Asn Trp Met Ser Ile Cys
        1395                1400                1405
Leu Tyr Gln Tyr Leu Lys Asp Ser Ala Gly Glu Pro Leu Tyr Lys Leu
    1410                1415                1420
Phe Lys Ala Ile Lys His Gln Val Glu Lys Gly Pro Val Asp Ala Val
1425                1430                1435                1440
Gln Lys Lys Ala Lys Tyr Thr Leu Asn Asp Thr Gly Leu Leu Gly Asp
                1445                1450                1455
Asp Val Glu Tyr Ala Pro Leu Thr Val Ser Val Ile Val Gln Asp Glu
            1460                1465                1470
Gly Val Asp Ala Ile Pro Val Lys Val Leu Asn Cys Asp Thr Ile Ser
        1475                1480                1485
Gln Val Lys Glu Lys Ile Ile Asp Gln Val Tyr Arg Gly Gln Pro Cys
    1490                1495                1500
Ser Cys Trp Pro Arg Pro Asp Ser Val Val Leu Glu Trp Arg Pro Gly
1505                1510                1515                1520
Ser Thr Ala Gln Ile Leu Ser Asp Leu Asp Leu Thr Ser Gln Arg Glu
                1525                1530                1535
Gly Arg Trp Lys Arg Val Asn Thr Leu Met His Tyr Asn Val Arg Asp
            1540                1545                1550
Gly Ala Thr Leu Ile Leu Ser Lys Val Gly Val Ser Gln Gln Pro Glu
        1555                1560                1565
Asp Ser Gln Gln Asp Leu Pro Gly Glu Arg His Ala Leu Leu Glu Glu
    1570                1575                1580
Glu Asn Arg Val Trp His Leu Val Arg Pro Thr Asp Glu Val Asp Glu
1585                1590                1595                1600
Gly Lys Ser Lys Arg Gly Ser Val Lys Glu Lys Glu Arg Thr Lys Ala
                1605                1610                1615
Ile Thr Glu Ile Tyr Leu Thr Arg Leu Leu Ser Val Lys Gly Thr Leu
            1620                1625                1630
Gln Gln Phe Val Asp Asn Phe Phe Gln Ser Val Leu Ala Pro Gly His
        1635                1640                1645
Ala Val Pro Pro Ala Val Lys Tyr Phe Phe Asp Phe Leu Asp Glu Gln
    1650                1655                1660
Ala Glu Lys His Asn Ile Gln Asp Glu Asp Thr Ile His Ile Trp Lys
1665                1670                1675                1680
Thr Asn Ser Leu Pro Leu Arg Phe Trp Val Asn Ile Leu Lys Asn Pro
                1685                1690                1695
His Phe Ile Phe Asp Val His Val His Glu Val Val Asp Ala Ser Leu
            1700                1705                1710
Ser Val Ile Ala Gln Thr Phe Met Asp Ala Cys Thr Arg Thr Glu His
        1715                1720                1725
Lys Leu Ser Arg Asp Ser Pro Ser Asn Lys Leu Leu Tyr Ala Lys Glu
    1730                1735                1740
Ile Ser Thr Tyr Lys Lys Met Val Glu Asp Tyr Tyr Lys Gly Ile Arg
1745                1750                1755                1760
Gln Met Val Gln Val Ser Asp Gln Asp Met Asn Thr His Leu Ala Glu
                1765                1770                1775
Ile Ser Arg Ala His Thr Asp Ser Leu Asn Thr Leu Val Ala Leu His
            1780                1785                1790
Gln Leu Tyr Gln Tyr Thr Gln Lys Tyr Tyr Asp Glu Ile Ile Asn Ala
        1795                1800                1805
Leu Glu Glu Asp Pro Ala Ala Gln Lys Met Gln Leu Ala Phe Arg Leu

1810                1815                1820
Gln Gln Ile Ala Ala Ala Leu Glu Asn Lys Val Thr Asp Leu
1825                1830                1835

<210> 5
<211> 5859
<212> DNA
<213> Homo sapiens

<220>
<223> PLXNB1-204 cDNA (coding sequence)

<400> 5
   atgcctgctc tgggcccagc tcttctccag gctctctggg ccgggtgggt cctcaccctc      60
   cagccccttc caccaactgc attcactccc aatggcacgt atctgcagca cctggcaagg     120
   gaccccacct caggcaccct ctacctgggg gctaccaact tcctgttcca gctgagccct     180
   gggctgcagc tggaggccac agtgtccacc ggccctgtgc tagacagcag ggactgcctg     240
   ccacctgtga tgcctgatga gtgcccccag gcccagccta ccaacaaccc gaatcagctg     300
   ctcctggtga gcccaggggc cctggtggta tgcgggagcg tgcaccaggg ggtctgtgaa     360
   cagcggcgcc tggggcagct cgagcagctg ctgctgcggc cagagcggcc tggggacaca     420
   caatatgtgg ctgccaatga tcctgcggtc agcacggtgg ggctggtagc ccagggcttg     480
   gcaggggagc cctcctgttt tgtggggcga ggatacacca gcaggggtgt ggggggtggc     540
   attccaccca tcacaacccg ggccctgtgg ccgcccgacc cccaagctgc cttctcctat     600
   gaggagacag ccaagctggc agtgggccgc ctctccgagt acagccacca cttcgtgagt     660
   gcctttgcac gtggggccag cgcctacttc ctgttcctgc ggcgggacct gcaggctcag     720
   tctagagctt ttcgtgccta tgtatctcga gtgtgtctcc gggaccagca ctactactcc     780
   tatgtggagt tgcctctggc ctgcgaaggt ggccgctacg ggctgatcca ggctgcagct     840
   gtggccacgt ccagggaggt ggcgcatggg gaggtgctct ttgcagcttt ctcctcggct     900
   gcacccccca ctgtgggccg gcccccatcg gcggctgctg gggcatctgg agcctctgcc     960
   ctctgtgcct tccccctgga tgaggtggac cggcttgcta atcgcacgcg agatgcctgc    1020
   tacacccggg agggtcgtgc tgaggatggg accgaggtgg cctacatcga gtatgatgtc    1080
   aattctgact gtgcacagct gccagtggac accctggatg cttatccctg tggctcagac    1140
   cacacgccca gccccatggc cagccgggtc ccgctggaag ccacaccaat tctggagtgg    1200
   ccagggattc agctaacagc tgtggcagtc accatggaag atggacacac catcgctttc    1260
   ctgggtgata gtcaagggca gctgcacagg gtctacttgg gcccagggag cgatggccac    1320
   ccatactcca cacagagcat ccagcagggg tctgcagtga gcagagacct cacctttgat    1380
   gggacctttg agcacctgta tgtcatgacc cagagcacac ttctgaaggt tcctgtggct    1440
   tcctgtgctc agcacctgga ctgtgcatct tgccttgctc acagggaccc atactgtggg    1500
   tggtgcgtgc tccttggcag gtgcagtcgc cgttctgagt gctcgagggg ccagggccca 1560
   gagcagtggc tatggagctt ccagcctgag ctgggctgtc tgcaagtggc agccatgagt 1620
   cctgccaaca tcagccgaga ggagacgagg gaggttttcc tatcagtgcc agacctgcca 1680
   cccctgtggc caggggagtc atattcctgc cactttgggg aacatcagag tcctgccctg 1740
   ctgactggtt ctggtgtgat gtgcccctcc ccagacccta gtgaggcccc agtgctgccg 1800
   agaggagccg actacgtatc cgtgagcgtg gagctcagat ttggcgctgt tgtgatcgcc 1860
   aaaacttccc tctctttcta tgactgtgtg gcggtcactg aactccgccc atctgcgcag 1920
   tgccaggcct gtgtgagcag ccgctggggg tgtaactggt gtgtctggca gcacctgtgc 1980
   acccacaagg cctcgtgtga tgctgggccc atggttgcaa gccatcagag cccgcttgtc 2040
   tccccagacc ctcctgcaag aggtgatgga gactcagcag agcttgaggg ccctcccgcc 2100
   cccctcatcc tcccgtccag cctcgactac cagtatgaca cccccgggct ctgggagctg 2160
   gaagaggcga ccttgggggc aagctcctgc ccctgtgtgg agagcgttca gggctccacg 2220
   ttgatgccgg tccatgtgga gcgggaaatc cggctgctag gcaggaacct gcaccttttc 2280
   caggatggcc caggagacaa tgagtgtgtg atggagctgg agggcctcga ggtggtggtt 2340
   gaggcccggg tcgagtgtga gccacctcca gatacccagt gccatgtcac ctgccagcag 2400
   caccagctca gctatgaggc tctgcagccg gagctccgtg tggggctgtt tctgcgtcgg 2460
   gccggccgtc tgcgtgtgga cagtgctgag gggctgcatg tggtactgta tgactgttcc 2520
   gtgggacatg gagactgcag ccgctgccaa actgccatgc cccagtatgg ctgtgtgtgg 2580
   tgtgaggggg agcgtccacg ttgtgtgacc cgggaggcct gtggtgaggc tgaggctgtg 2640
   gccacccagt gcccagcgcc cctcatccac tcggtggagc cactgactgg gcctgtagac 2700
   ggaggcaccc gtgtcaccat caggggctcc aacctgggcc agcatgtgca ggatgtgctg 2760
   ggcatggtca cggtggctgg agtgccctgt gctgtggatg cccaggagta cgaggtctcc 2820
   agcagcctcg tgtgcatcac cggggccagt ggggaggagg tggccggcgc cacagcggtg 2880
   gaggtgccgg gaagaggacg tggtgtctca gaacacgact ttgcctacca ggatccgaag 2940
   gtccattcca tcttcccggc ccgcggcccc agagctgggg gcacccgtct caccctgaat 3000
   ggctccaagc tcctgactgg gcggctggag gacatccgag tggtggttgg agaccagcct 3060
   tgtcacttgc tgccggagca gcagtcagaa caactgcggt gtgagaccag cccacgcccc 3120
   acgcctgcca cgctccctgt ggctgtgtgg tttggggcca cggagcggag gcttcaacgc 3180
   ggacagttca agtatacctt ggaccccaac atcacctctg ctggccccac caagagcttc 3240
   ctcagtggag gacgtgagat atgcgtccgt ggccagaatc tggacgtggt acagacgcca 3300
   agaatccggg tgaccgtggt ctcgagaatg ctgcagccca gccaggggct tggacggagg 3360
   cgtcgcgtgg tcccggagac ggcatgttcc cttggaccct cctgcagtag ccagcaattt 3420
   gaggagccgt gccatgtcaa ctcctcccag ctcatcacgt gccgcacacc tgccctccca 3480
   ggcctgcctg aggacccctg ggtccgggtg gaatttatcc ttgacaacct ggtctttgac 3540
   tttgcaacac tgaaccccac acctttctcc tatgaggccg accccaccct gcagccactc 3600
   aaccctgagg accccaccat gccattccgg cacaagcctg ggagtgtgtt ctccgtggag 3660
   ggggagaacc tggaccttgc aatgtccaag gaggaggtgg tggctatgat aggggatggc 3720
   ccctgtgtgg tgaagacgct gacgcggcac cacctgtact gcgagccccc cgtggagcag 3780
   cccctgccac ggcaccatgc cctccgagag gcacctgact ctttgcctga gttcacggtg 3840
   cagatgggga acttgcgctt ctccctgggt cacgtgcagt atgacggcga gagccctggg 3900
   gcttttcctg tggcagccca ggtgggcttg ggggtgggca cctctcttct ggctctgggt 3960
   gtcatcatca ttgtcctcat gtacaggagg aagagcaagc aggccctgag ggactataag 4020
   aaggttcaga tccagctgga gaatctggag agcagtgtgc gggaccgctg caagaaggaa 4080
   ttcacagacc tcatgactga gatgaccgat ctcaccagtg acctcctggg cagcggcatc 4140
   cccttcctcg actacaaggt gtatgcggag aggatcttct tccctgggca ccgcgagtcg 4200
   cccttgcacc gggacctggg tgtgcctgag agcagacggc ccactgtgga gcaagggctg 4260
   gggcagctct ctaacctgct caacagcaag ctcttcctca ccaagttcat ccacacgctg 4320
   gagagccagc gcaccttttc agctcgggac cgtgcctacg tggcatctct gctcaccgtg 4380
   gcactgcatg ggaagcttga gtatttcact gacatcctcc gcactctgct cagtgacctg 4440
   gttgcccagt atgtggccaa gaaccccaag ctgatgctgc gcaggacaga gactgtggtg 4500
   gagaagctgc tcaccaactg gatgtccatc tgtctgtata ccttcgtgag ggactccgta 4560
   ggggagcctc tgtacatgct ctttcgaggg attaagcacc aagtggataa ggggccagtg 4620
   gacagtgtga caggcaaggc caaatacacc ttgaacgaca accgcctgct cagagaggat 4680
   gtggagtacc gtcccctgac cttgaatgca ctattggctg tggggcctgg ggcaggagag 4740
   gcccagggcg tgcccgtgaa ggtcctagac tgtgacacca tctcccaggc aaaggagaag 4800
   atgctggacc agctttataa aggagtgcct ctcacccagc ggccagaccc tcgcaccctt 4860
   gatgttgagt ggcggtctgg ggtggccggg cacctcattc tttctgacga ggatgtcact 4920
   tctgaggtcc agggtctgtg gaggcgcctg aacacactgc agcattacaa ggtcccagat 4980
   ggagcaactg tggccctcgt cccctgcctc accaagcatg tgctccggga aaaccaggat 5040
   tatgtccctg gagagcggac cccaatgctg gaggatgtag atgagggggg catccggccc 5100
   tggcacctgg tgaagccaag tgatgagccg gagccgccca ggcctcggag gggcagcctt 5160
   cggggcgggg agcgtgagcg cgccaaggcc atccctgaga tctacctgac ccgcctgctg 5220
   tccatgaagg gcaccctgca gaagttcgtg gatgacctgt tccaggtgat tctcagcacc 5280
   agccgccccg tgccgctcgc tgtgaagtac ttctttgacc tgctggatga gcaggcccag     5340
   cagcatggca tctccgacca ggacaccatc cacatctgga agaccaacag cttgcctctg     5400
   aggttctgga tcaatataat aaaaaacccg cagtttgtgt tcgacgtgca aacatctgat     5460
   aacatggatg cggtgctcct tgtcattgca cagaccttca tggacgcctg caccctggcc     5520
   gaccacaagc tgggccggga ctccccgatc aacaaacttc tgtatgcacg ggacattccc     5580
   cggtacaagc ggatggtgga aaggtactat gcagacatca gacagactgt cccagccagc     5640
   gaccaagaga tgaactctgt cctggctgaa ctgtcctgga actactccgg agacctcggg     5700
   gcgcgagtgg ccctgcatga actctacaag tacatcaaca agtactatga ccagatcatc     5760
   actgccctgg aggaggatgg cacggcccag aagatgcagc tgggctatcg gctccagcag     5820
   attgcagctg ctgtggaaaa caaggtcaca gatctatag                            5859

<210> 6
<211> 1952
<212> PRT
<213> Homo sapiens

<220>
<223> PLXNB1-204 protein

<400> 6
Met Pro Ala Leu Gly Pro Ala Leu Leu Gln Ala Leu Trp Ala Gly Trp
1               5                   10                  15
Val Leu Thr Leu Gln Pro Leu Pro Pro Thr Ala Phe Thr Pro Asn Gly
            20                  25                  30
Thr Tyr Leu Gln His Leu Ala Arg Asp Pro Thr Ser Gly Thr Leu Tyr
        35                  40                  45
Leu Gly Ala Thr Asn Phe Leu Phe Gln Leu Ser Pro Gly Leu Gln Leu
    50                  55                  60
Glu Ala Thr Val Ser Thr Gly Pro Val Leu Asp Ser Arg Asp Cys Leu
65                  70                  75                  80
Pro Pro Val Met Pro Asp Glu Cys Pro Gln Ala Gln Pro Thr Asn Asn
                85                  90                  95
Pro Asn Gln Leu Leu Leu Val Ser Pro Gly Ala Leu Val Val Cys Gly
            100                 105                 110
Ser Val His Gln Gly Val Cys Glu Gln Arg Arg Leu Gly Gln Leu Glu
        115                 120                 125
Gln Leu Leu Leu Arg Pro Glu Arg Pro Gly Asp Thr Gln Tyr Val Ala
    130                 135                 140
Ala Asn Asp Pro Ala Val Ser Thr Val Gly Leu Val Ala Gln Gly Leu
145                 150                 155                 160
Ala Gly Glu Pro Leu Leu Phe Val Gly Arg Gly Tyr Thr Ser Arg Gly
                165                 170                 175
Val Gly Gly Gly Ile Pro Pro Ile Thr Thr Arg Ala Leu Trp Pro Pro
            180                 185                 190
Asp Pro Gln Ala Ala Phe Ser Tyr Glu Glu Thr Ala Lys Leu Ala Val
        195                 200                 205
Gly Arg Leu Ser Glu Tyr Ser His His Phe Val Ser Ala Phe Ala Arg
    210                 215                 220
Gly Ala Ser Ala Tyr Phe Leu Phe Leu Arg Arg Asp Leu Gln Ala Gln
225                 230                 235                 240
Ser Arg Ala Phe Arg Ala Tyr Val Ser Arg Val Cys Leu Arg Asp Gln
                245                 250                 255
His Tyr Tyr Ser Tyr Val Glu Leu Pro Leu Ala Cys Glu Gly Gly Arg

            260                 265                 270
Tyr Gly Leu Ile Gln Ala Ala Ala Val Ala Thr Ser Arg Glu Val Ala
        275                 280                 285
His Gly Glu Val Leu Phe Ala Ala Phe Ser Ser Ala Ala Pro Pro Thr
    290                 295                 300
Val Gly Arg Pro Pro Ser Ala Ala Ala Gly Ala Ser Gly Ala Ser Ala
305                 310                 315                 320
Leu Cys Ala Phe Pro Leu Asp Glu Val Asp Arg Leu Ala Asn Arg Thr
                325                 330                 335
Arg Asp Ala Cys Tyr Thr Arg Glu Gly Arg Ala Glu Asp Gly Thr Glu
            340                 345                 350
Val Ala Tyr Ile Glu Tyr Asp Val Asn Ser Asp Cys Ala Gln Leu Pro
        355                 360                 365
Val Asp Thr Leu Asp Ala Tyr Pro Cys Gly Ser Asp His Thr Pro Ser
    370                 375                 380
Pro Met Ala Ser Arg Val Pro Leu Glu Ala Thr Pro Ile Leu Glu Trp
385                 390                 395                 400
Pro Gly Ile Gln Leu Thr Ala Val Ala Val Thr Met Glu Asp Gly His
                405                 410                 415
Thr Ile Ala Phe Leu Gly Asp Ser Gln Gly Gln Leu His Arg Val Tyr
            420                 425                 430
Leu Gly Pro Gly Ser Asp Gly His Pro Tyr Ser Thr Gln Ser Ile Gln
        435                 440                 445
Gln Gly Ser Ala Val Ser Arg Asp Leu Thr Phe Asp Gly Thr Phe Glu
    450                 455                 460
His Leu Tyr Val Met Thr Gln Ser Thr Leu Leu Lys Val Pro Val Ala
465                 470                 475                 480
Ser Cys Ala Gln His Leu Asp Cys Ala Ser Cys Leu Ala His Arg Asp
                485                 490                 495
Pro Tyr Cys Gly Trp Cys Val Leu Leu Gly Arg Cys Ser Arg Arg Ser
            500                 505                 510
Glu Cys Ser Arg Gly Gln Gly Pro Glu Gln Trp Leu Trp Ser Phe Gln
        515                 520                 525
Pro Glu Leu Gly Cys Leu Gln Val Ala Ala Met Ser Pro Ala Asn Ile
    530                 535                 540
Ser Arg Glu Glu Thr Arg Glu Val Phe Leu Ser Val Pro Asp Leu Pro
545                 550                 555                 560
Pro Leu Trp Pro Gly Glu Ser Tyr Ser Cys His Phe Gly Glu His Gln
                565                 570                 575
Ser Pro Ala Leu Leu Thr Gly Ser Gly Val Met Cys Pro Ser Pro Asp
            580                 585                 590
Pro Ser Glu Ala Pro Val Leu Pro Arg Gly Ala Asp Tyr Val Ser Val
        595                 600                 605
Ser Val Glu Leu Arg Phe Gly Ala Val Val Ile Ala Lys Thr Ser Leu
    610                 615                 620
Ser Phe Tyr Asp Cys Val Ala Val Thr Glu Leu Arg Pro Ser Ala Gln
625                 630                 635                 640
Cys Gln Ala Cys Val Ser Ser Arg Trp Gly Cys Asn Trp Cys Val Trp
                645                 650                 655
Gln His Leu Cys Thr His Lys Ala Ser Cys Asp Ala Gly Pro Met Val
            660                 665                 670
Ala Ser His Gln Ser Pro Leu Val Ser Pro Asp Pro Pro Ala Arg Gly
        675                 680                 685
Asp Gly Asp Ser Ala Glu Leu Glu Gly Pro Pro Ala Pro Leu Ile Leu
    690                 695                 700
Pro Ser Ser Leu Asp Tyr Gln Tyr Asp Thr Pro Gly Leu Trp Glu Leu
705                 710                 715                 720
Glu Glu Ala Thr Leu Gly Ala Ser Ser Cys Pro Cys Val Glu Ser Val
                725                 730                 735
Gln Gly Ser Thr Leu Met Pro Val His Val Glu Arg Glu Ile Arg Leu
            740                 745                 750
Leu Gly Arg Asn Leu His Leu Phe Gln Asp Gly Pro Gly Asp Asn Glu
        755                 760                 765

Cys Val Met Glu Leu Glu Gly Leu Glu Val Val Val Glu Ala Arg Val
    770                 775                 780
Glu Cys Glu Pro Pro Pro Asp Thr Gln Cys His Val Thr Cys Gln Gln
785                 790                 795                 800
His Gln Leu Ser Tyr Glu Ala Leu Gln Pro Glu Leu Arg Val Gly Leu
                805                 810                 815
Phe Leu Arg Arg Ala Gly Arg Leu Arg Val Asp Ser Ala Glu Gly Leu
            820                 825                 830
His Val Val Leu Tyr Asp Cys Ser Val Gly His Gly Asp Cys Ser Arg
        835                 840                 845
Cys Gln Thr Ala Met Pro Gln Tyr Gly Cys Val Trp Cys Glu Gly Glu
    850                 855                 860
Arg Pro Arg Cys Val Thr Arg Glu Ala Cys Gly Glu Ala Glu Ala Val
865                 870                 875                 880
Ala Thr Gln Cys Pro Ala Pro Leu Ile His Ser Val Glu Pro Leu Thr
                885                 890                 895
Gly Pro Val Asp Gly Gly Thr Arg Val Thr Ile Arg Gly Ser Asn Leu
            900                 905                 910
Gly Gln His Val Gln Asp Val Leu Gly Met Val Thr Val Ala Gly Val
        915                 920                 925
Pro Cys Ala Val Asp Ala Gln Glu Tyr Glu Val Ser Ser Ser Leu Val
    930                 935                 940
Cys Ile Thr Gly Ala Ser Gly Glu Glu Val Ala Gly Ala Thr Ala Val
945                 950                 955                 960
Glu Val Pro Gly Arg Gly Arg Gly Val Ser Glu His Asp Phe Ala Tyr
                965                 970                 975
Gln Asp Pro Lys Val His Ser Ile Phe Pro Ala Arg Gly Pro Arg Ala
            980                 985                 990
Gly Gly Thr Arg Leu Thr Leu Asn Gly Ser Lys Leu Leu Thr Gly Arg
        995                 1000                1005
Leu Glu Asp Ile Arg Val Val Val Gly Asp Gln Pro Cys His Leu Leu
    1010                1015                1020
Pro Glu Gln Gln Ser Glu Gln Leu Arg Cys Glu Thr Ser Pro Arg Pro
1025                1030                1035                1040
Thr Pro Ala Thr Leu Pro Val Ala Val Trp Phe Gly Ala Thr Glu Arg
                1045                1050                1055
Arg Leu Gln Arg Gly Gln Phe Lys Tyr Thr Leu Asp Pro Asn Ile Thr
            1060                1065                1070
Ser Ala Gly Pro Thr Lys Ser Phe Leu Ser Gly Gly Arg Glu Ile Cys
        1075                1080                1085
Val Arg Gly Gln Asn Leu Asp Val Val Gln Thr Pro Arg Ile Arg Val
    1090                1095                1100
Thr Val Val Ser Arg Met Leu Gln Pro Ser Gln Gly Leu Gly Arg Arg
1105                1110                1115                1120
Arg Arg Val Val Pro Glu Thr Ala Cys Ser Leu Gly Pro Ser Cys Ser
                1125                1130                1135
Ser Gln Gln Phe Glu Glu Pro Cys His Val Asn Ser Ser Gln Leu Ile
            1140                1145                1150
Thr Cys Arg Thr Pro Ala Leu Pro Gly Leu Pro Glu Asp Pro Trp Val
        1155                1160                1165
Arg Val Glu Phe Ile Leu Asp Asn Leu Val Phe Asp Phe Ala Thr Leu
    1170                1175                1180
Asn Pro Thr Pro Phe Ser Tyr Glu Ala Asp Pro Thr Leu Gln Pro Leu
1185                1190                1195                1200
Asn Pro Glu Asp Pro Thr Met Pro Phe Arg His Lys Pro Gly Ser Val
                1205                1210                1215
Phe Ser Val Glu Gly Glu Asn Leu Asp Leu Ala Met Ser Lys Glu Glu
            1220                1225                1230
Val Val Ala Met Ile Gly Asp Gly Pro Cys Val Val Lys Thr Leu Thr
        1235                1240                1245
Arg His His Leu Tyr Cys Glu Pro Pro Val Glu Gln Pro Leu Pro Arg
    1250                1255                1260
His His Ala Leu Arg Glu Ala Pro Asp Ser Leu Pro Glu Phe Thr Val

1265                1270                1275                1280
Gln Met Gly Asn Leu Arg Phe Ser Leu Gly His Val Gln Tyr Asp Gly
                1285                1290                1295
Glu Ser Pro Gly Ala Phe Pro Val Ala Ala Gln Val Gly Leu Gly Val
            1300                1305                1310
Gly Thr Ser Leu Leu Ala Leu Gly Val Ile Ile Ile Val Leu Met Tyr
        1315                1320                1325
Arg Arg Lys Ser Lys Gln Ala Leu Arg Asp Tyr Lys Lys Val Gln Ile
    1330                1335                1340
Gln Leu Glu Asn Leu Glu Ser Ser Val Arg Asp Arg Cys Lys Lys Glu
1345                1350                1355                1360
Phe Thr Asp Leu Met Thr Glu Met Thr Asp Leu Thr Ser Asp Leu Leu
                1365                1370                1375
Gly Ser Gly Ile Pro Phe Leu Asp Tyr Lys Val Tyr Ala Glu Arg Ile
            1380                1385                1390
Phe Phe Pro Gly His Arg Glu Ser Pro Leu His Arg Asp Leu Gly Val
        1395                1400                1405
Pro Glu Ser Arg Arg Pro Thr Val Glu Gln Gly Leu Gly Gln Leu Ser
    1410                1415                1420
Asn Leu Leu Asn Ser Lys Leu Phe Leu Thr Lys Phe Ile His Thr Leu
1425                1430                1435                1440
Glu Ser Gln Arg Thr Phe Ser Ala Arg Asp Arg Ala Tyr Val Ala Ser
                1445                1450                1455
Leu Leu Thr Val Ala Leu His Gly Lys Leu Glu Tyr Phe Thr Asp Ile
            1460                1465                1470
Leu Arg Thr Leu Leu Ser Asp Leu Val Ala Gln Tyr Val Ala Lys Asn
        1475                1480                1485
Pro Lys Leu Met Leu Arg Arg Thr Glu Thr Val Val Glu Lys Leu Leu
    1490                1495                1500
Thr Asn Trp Met Ser Ile Cys Leu Tyr Thr Phe Val Arg Asp Ser Val
1505                1510                1515                1520
Gly Glu Pro Leu Tyr Met Leu Phe Arg Gly Ile Lys His Gln Val Asp
                1525                1530                1535
Lys Gly Pro Val Asp Ser Val Thr Gly Lys Ala Lys Tyr Thr Leu Asn
            1540                1545                1550
Asp Asn Arg Leu Leu Arg Glu Asp Val Glu Tyr Arg Pro Leu Thr Leu
        1555                1560                1565
Asn Ala Leu Leu Ala Val Gly Pro Gly Ala Gly Glu Ala Gln Gly Val
    1570                1575                1580
Pro Val Lys Val Leu Asp Cys Asp Thr Ile Ser Gln Ala Lys Glu Lys
1585                1590                1595                1600
Met Leu Asp Gln Leu Tyr Lys Gly Val Pro Leu Thr Gln Arg Pro Asp
                1605                1610                1615
Pro Arg Thr Leu Asp Val Glu Trp Arg Ser Gly Val Ala Gly His Leu
            1620                1625                1630
Ile Leu Ser Asp Glu Asp Val Thr Ser Glu Val Gln Gly Leu Trp Arg
        1635                1640                1645
Arg Leu Asn Thr Leu Gln His Tyr Lys Val Pro Asp Gly Ala Thr Val
    1650                1655                1660
Ala Leu Val Pro Cys Leu Thr Lys His Val Leu Arg Glu Asn Gln Asp
1665                1670                1675                1680
Tyr Val Pro Gly Glu Arg Thr Pro Met Leu Glu Asp Val Asp Glu Gly
                1685                1690                1695
Gly Ile Arg Pro Trp His Leu Val Lys Pro Ser Asp Glu Pro Glu Pro
            1700                1705                1710
Pro Arg Pro Arg Arg Gly Ser Leu Arg Gly Gly Glu Arg Glu Arg Ala
        1715                1720                1725
Lys Ala Ile Pro Glu Ile Tyr Leu Thr Arg Leu Leu Ser Met Lys Gly
    1730                1735                1740
Thr Leu Gln Lys Phe Val Asp Asp Leu Phe Gln Val Ile Leu Ser Thr
1745                1750                1755                1760
Ser Arg Pro Val Pro Leu Ala Val Lys Tyr Phe Phe Asp Leu Leu Asp
                1765                1770                1775

Glu Gln Ala Gln Gln His Gly Ile Ser Asp Gln Asp Thr Ile His Ile
            1780                1785                1790
Trp Lys Thr Asn Ser Leu Pro Leu Arg Phe Trp Ile Asn Ile Ile Lys
        1795                1800                1805
Asn Pro Gln Phe Val Phe Asp Val Gln Thr Ser Asp Asn Met Asp Ala
    1810                1815                1820
Val Leu Leu Val Ile Ala Gln Thr Phe Met Asp Ala Cys Thr Leu Ala
1825                1830                1835                1840
Asp His Lys Leu Gly Arg Asp Ser Pro Ile Asn Lys Leu Leu Tyr Ala
                1845                1850                1855
Arg Asp Ile Pro Arg Tyr Lys Arg Met Val Glu Arg Tyr Tyr Ala Asp
            1860                1865                1870
Ile Arg Gln Thr Val Pro Ala Ser Asp Gln Glu Met Asn Ser Val Leu
        1875                1880                1885
Ala Glu Leu Ser Trp Asn Tyr Ser Gly Asp Leu Gly Ala Arg Val Ala
    1890                1895                1900
Leu His Glu Leu Tyr Lys Tyr Ile Asn Lys Tyr Tyr Asp Gln Ile Ile
1905                1910                1915                1920
Thr Ala Leu Glu Glu Asp Gly Thr Ala Gln Lys Met Gln Leu Gly Tyr
                1925                1930                1935
Arg Leu Gln Gln Ile Ala Ala Ala Val Glu Asn Lys Val Thr Asp Leu
            1940                1945                1950

<210> 7
<211> 704
<212> DNA
<213> Homo sapiens

<220>
<223> PLXNB2-202 cDNA (coding sequence)

<400> 7
tacaagccct tccacggcga tatccagtgc ggcggccacg cgccgggctc cagcaagagc      60

ttcccatgtg gctcggagca cctgccctac ccgctgggca gccgcgacgg gctcagaggc     120

acagccgtgc tgcagcgtgg aggcctgaac ctcacggccg tgacggtcgc cgccgagaac     180

aaccacactg ttgcttttct ggagtggcgt ccgggctcca cagcgcagat cctgtcggac     240

ctggacctga cgtcacagcg ggagggccgg tggaagcgcg tcaacaccct tatgcactac     300

aatgtccggg atggagccac cctcatcctg tccaaggtgg gggtctccca gcagccggag     360

gacagccagc aggacctgcc tggggagcgc catgccctcc tggaggagga gaaccgggtg     420

tggcacctgg tgcggccgac cgacgaggtg gacgagggca agtccaagag aggcagcgtg     480

aaagagaagg agcggacgaa ggccatcacc gagatctacc tgacgcggct gctctcagtc     540

aagggcacac tgcagcagtt tgtggacaac ttcttccaga gcgtgctggc gcctgggcac     600

gcggtgccac ctgcagtcaa gtacttcttc gacttcctgg acgagcaggc agagaagcac     660

aacatccagg atgaagacac catccacatc tggaagacga acag                      704

<210> 8
<211> 234
<212> PRT
<213> Homo sapiens

<220>
<223> PLXNB2-202 protein

<400> 8
Tyr Lys Pro Phe His Gly Asp Ile Gln Cys Gly Gly His Ala Pro Gly
1               5                   10                  15
Ser Ser Lys Ser Phe Pro Cys Gly Ser Glu His Leu Pro Tyr Pro Leu
            20                  25                  30
Gly Ser Arg Asp Gly Leu Arg Gly Thr Ala Val Leu Gln Arg Gly Gly
        35                  40                  45
Leu Asn Leu Thr Ala Val Thr Val Ala Ala Glu Asn Asn His Thr Val
    50                  55                  60
Ala Phe Leu Glu Trp Arg Pro Gly Ser Thr Ala Gln Ile Leu Ser Asp
65                  70                  75                  80
Leu Asp Leu Thr Ser Gln Arg Glu Gly Arg Trp Lys Arg Val Asn Thr
                85                  90                  95
Leu Met His Tyr Asn Val Arg Asp Gly Ala Thr Leu Ile Leu Ser Lys
            100                 105                 110
Val Gly Val Ser Gln Gln Pro Glu Asp Ser Gln Gln Asp Leu Pro Gly
        115                 120                 125
Glu Arg His Ala Leu Leu Glu Glu Glu Asn Arg Val Trp His Leu Val
    130                 135                 140
Arg Pro Thr Asp Glu Val Asp Glu Gly Lys Ser Lys Arg Gly Ser Val
145                 150                 155                 160
Lys Glu Lys Glu Arg Thr Lys Ala Ile Thr Glu Ile Tyr Leu Thr Arg
                165                 170                 175
Leu Leu Ser Val Lys Gly Thr Leu Gln Gln Phe Val Asp Asn Phe Phe
            180                 185                 190
Gln Ser Val Leu Ala Pro Gly His Ala Val Pro Pro Ala Val Lys Tyr
        195                 200                 205
Phe Phe Asp Phe Leu Asp Glu Gln Ala Glu Lys His Asn Ile Gln Asp
    210                 215                 220
Glu Asp Thr Ile His Ile Trp Lys Thr Asn
225                 230

<210> 9
<211> 485
<212> DNA
<213> Homo sapiens

<220>
<223> PLXNB2-203 cDNA (coding sequence)

<400> 9
atggcactgc agctctgggc cctgaccctg ctgggcctgc tgggcgcagg tgccagcctg      60

aggccccgca agctggactt cttccgcagc gagaaagagc tgaaccacct ggctgtggat     120

gaggcctcag gcgtggtgta cctgggggcg gtgaatgccc tctaccagct ggatgcgaag     180

ctgcagctgg agcagcaggt ggccacgggc ccggccctgg acaacaagaa gtgcacgccg     240

cccatcgagg ccagccagtg ccatgaggct gagatgactg acaatgtcaa ccagctgctg     300

ctgctcgacc ctcccaggaa gcgcctggtg gagtgcggca gcctcttcaa gggcatctgc     360

gctctgcgcg ccctgagcaa catctccctc cgcctgttct acgaggacgg cagcggggag     420

aagtctttcg tggccagcaa tgatgagggc gtggccacag tggggctggt gagctccacg     480

ggtcc                                                                 485

<210> 10
<211> 161
<212> PRT
<213> Homo sapiens

<220>
<223> PLXNB2-203 protein

<400> 10
Met Ala Leu Gln Leu Trp Ala Leu Thr Leu Leu Gly Leu Leu Gly Ala
1               5                   10                  15
Gly Ala Ser Leu Arg Pro Arg Lys Leu Asp Phe Phe Arg Ser Glu Lys
            20                  25                  30
Glu Leu Asn His Leu Ala Val Asp Glu Ala Ser Gly Val Val Tyr Leu
        35                  40                  45
Gly Ala Val Asn Ala Leu Tyr Gln Leu Asp Ala Lys Leu Gln Leu Glu
    50                  55                  60
Gln Gln Val Ala Thr Gly Pro Ala Leu Asp Asn Lys Lys Cys Thr Pro
65                  70                  75                  80
Pro Ile Glu Ala Ser Gln Cys His Glu Ala Glu Met Thr Asp Asn Val
                85                  90                  95
Asn Gln Leu Leu Leu Leu Asp Pro Pro Arg Lys Arg Leu Val Glu Cys
            100                 105                 110
Gly Ser Leu Phe Lys Gly Ile Cys Ala Leu Arg Ala Leu Ser Asn Ile
        115                 120                 125
Ser Leu Arg Leu Phe Tyr Glu Asp Gly Ser Gly Glu Lys Ser Phe Val
    130                 135                 140
Ala Ser Asn Asp Glu Gly Val Ala Thr Val Gly Leu Val Ser Ser Thr
145                 150                 155                 160
Gly


<210> 11
<211> 568
<212> DNA
<213> Homo sapiens

<220>
<223> PLXNB2-204 cDNA (coding sequence)

<220>
<221> variation
<222> 1
<223> /replace="a,c,g,t"

<400> 11
   nacgaagttt ggggcgcagc tccagtgtgt cactggcccc caggcgacac ggggccagat      60
   gcttctggag gtctcctacg gggggtcccc cgtgcccaac cccggcatct tcttcaccta     120
   ccgcgaaaac cccgtactgc gagccttcga gccgctacga agctttgcca ggtggtgggt     180
   acagactacg tgttccacaa tgacaccaag gtcgtcttcc tgtccccggc tgtgcctgag     240
   gagccagagg cctacaacct cacggtgctg atcgagatgg acgggcaccg tgccctgctc     300
   agaacagagg ccggggcctt cgagtacgtg cctgacccca cctttgagaa cttcacaggt     360
   ggcgtcaaga agcaggtcaa caagctcatc cacgcccggg gcaccaatct gaacaaggcg     420
   atgacgctgc aggaggccga ggccttcgtg ggtgccgagc gctgcaccat gaagacgctg     480
   acggagaccg acctgtactg tgagcccccg gaggtgcagc ccccgcccaa gcggcggcag 540
   aaacgagaca ccacacacaa cctgcccg 568

<210> 12
<211> 189
<212> PRT
<213> Homo sapiens

<220>
<223> PLXNB2-204 protein

<220>
<221> VARIANT
<222> 1
<223> Ala,Cys,Asp,Glu,Phe,Gly,His,Ile,Lys,Leu,Met,Asn,Pro,Gln,Arg,Ser,T
      hr,Val,Trp,Tyr

<400> 12
Xaa Glu Val Trp Gly Ala Ala Pro Val Cys His Trp Pro Pro Gly Asp
1               5                   10                  15
Thr Gly Pro Asp Ala Ser Gly Gly Leu Leu Arg Gly Val Pro Arg Ala
            20                  25                  30
Gln Pro Arg His Leu Leu His Leu Pro Arg Lys Pro Arg Thr Ala Ser
        35                  40                  45
Leu Arg Ala Ala Thr Lys Leu Cys Gln Val Val Gly Thr Asp Tyr Val
    50                  55                  60
Phe His Asn Asp Thr Lys Val Val Phe Leu Ser Pro Ala Val Pro Glu
65                  70                  75                  80
Glu Pro Glu Ala Tyr Asn Leu Thr Val Leu Ile Glu Met Asp Gly His
                85                  90                  95
Arg Ala Leu Leu Arg Thr Glu Ala Gly Ala Phe Glu Tyr Val Pro Asp
            100                 105                 110
Pro Thr Phe Glu Asn Phe Thr Gly Gly Val Lys Lys Gln Val Asn Lys
        115                 120                 125
Leu Ile His Ala Arg Gly Thr Asn Leu Asn Lys Ala Met Thr Leu Gln
    130                 135                 140
Glu Ala Glu Ala Phe Val Gly Ala Glu Arg Cys Thr Met Lys Thr Leu
145                 150                 155                 160
Thr Glu Thr Asp Leu Tyr Cys Glu Pro Pro Glu Val Gln Pro Pro Pro
                165                 170                 175
Lys Arg Arg Gln Lys Arg Asp Thr Thr His Asn Leu Pro
            180                 185

<210> 13
<211> 1431
<212> DNA
<213> Homo sapiens

<220>
<223> PLXNB2-205 cDNA (coding sequence

<400> 13
atggcactgc agctctgggc cctgaccctg ctgggcctgc tgggcgcagg tgccagcctg 60

aggccccgca agctggactt cttccgcagc gagaaagagc tgaaccacct ggctgtggat 120

gaggcctcag gcgtggtgta cctgggggcg gtgaatgccc tctaccagct ggatgcgaag 180

ctgcagctgg agcagcaggt ggccacgggc ccggccctgg acaacaagaa gtgcacgccg 240

cccatcgagg ccagccagtg ccatgaggct gagatgactg acaatgtcaa ccagctgctg 300

ctgctcgacc ctcccaggaa gcgcctggtg gagtgcggca gcctcttcaa gggcatctgc 360

gctctgcgcg ccctgagcaa catctccctc cgcctgttct acgaggacgg cagcggggag 420

aagtctttcg tggccagcaa tgatgagggc gtggccacag tggggctggt gagctccacg 480

ggtcctggtg gtgaccgcgt gctgtttgtg ggcaaaggca atgggccaca cgacaacggc 540

atcatcgtga gcactcggct gttggaccgg actgacagca gggaggcctt tgaagcctac 600

acggaccacg ccacctacaa ggccggctac ctgtccacca acacacagca gttcgtggcg 660

gccttcgagg acggccccta cgtcttcttt gtcttcaacc agcaggacaa gcacccggcc 720

cggaaccgca cgctgctggc acgcatgtgc agagaagacc ccaactacta ctcctacctg 780

gagatggacc tgcagtgccg ggaccccgac atccacgccg ctgcctttgg cacctgcctg 840

gccgcctccg tggctgcgcc tggctctggc agggtgctat atgctgtctt cagcagagac 900

agccggagca gtgggggccc cggtgcgggc ctctgcctgt tcccgctgga caaggtgcac 960

gccaagatgg aggccaaccg caacgcctgt tacacaggca cccgggaggc ccgtgacatc 1020

ttctacaagc ccttccacgg cgatatccag tgcggcggcc acgcgccggg ctccagcaag 1080

agcttcccat gtggctcgga gcacctgccc tacccgctgg gcagccgcga cgggctcaga 1140

ggcacagccg tgctgcagcg tggaggcctg aacctcacgg ccgtgacggt cgccgccgag 1200

aacaaccaca ctgttgcttt tctgggcacc tctgatggcc ggatcctcaa ggtgtacctc 1260

accccagatg gcacctcctc agagtacgac tctatccttg tggagataaa caagagagtc 1320

aagcgcgacc tggtactgtc tggagacctg ggcagcctgt acgccatgac ccaggacaag 1380

gtgttccggc tgccggtgca ggagtgcctg agctacccga cctgcaccca g 1431

<210> 14
<211> 477
<212> PRT
<213> Homo sapiens

<220>
<223> PLXNB2-205 protein

<400> 14
Met Ala Leu Gln Leu Trp Ala Leu Thr Leu Leu Gly Leu Leu Gly Ala
1               5                   10                  15
Gly Ala Ser Leu Arg Pro Arg Lys Leu Asp Phe Phe Arg Ser Glu Lys
            20                  25                  30
Glu Leu Asn His Leu Ala Val Asp Glu Ala Ser Gly Val Val Tyr Leu
        35                  40                  45
Gly Ala Val Asn Ala Leu Tyr Gln Leu Asp Ala Lys Leu Gln Leu Glu
    50                  55                  60
Gln Gln Val Ala Thr Gly Pro Ala Leu Asp Asn Lys Lys Cys Thr Pro
65                  70                  75                  80
Pro Ile Glu Ala Ser Gln Cys His Glu Ala Glu Met Thr Asp Asn Val
                85                  90                  95
Asn Gln Leu Leu Leu Leu Asp Pro Pro Arg Lys Arg Leu Val Glu Cys
            100                 105                 110

Gly Ser Leu Phe Lys Gly Ile Cys Ala Leu Arg Ala Leu Ser Asn Ile
        115                 120                 125
Ser Leu Arg Leu Phe Tyr Glu Asp Gly Ser Gly Glu Lys Ser Phe Val
    130                 135                 140
Ala Ser Asn Asp Glu Gly Val Ala Thr Val Gly Leu Val Ser Ser Thr
145                 150                 155                 160
Gly Pro Gly Gly Asp Arg Val Leu Phe Val Gly Lys Gly Asn Gly Pro
                165                 170                 175
His Asp Asn Gly Ile Ile Val Ser Thr Arg Leu Leu Asp Arg Thr Asp
            180                 185                 190
Ser Arg Glu Ala Phe Glu Ala Tyr Thr Asp His Ala Thr Tyr Lys Ala
        195                 200                 205
Gly Tyr Leu Ser Thr Asn Thr Gln Gln Phe Val Ala Ala Phe Glu Asp
    210                 215                 220
Gly Pro Tyr Val Phe Phe Val Phe Asn Gln Gln Asp Lys His Pro Ala
225                 230                 235                 240
Arg Asn Arg Thr Leu Leu Ala Arg Met Cys Arg Glu Asp Pro Asn Tyr
                245                 250                 255
Tyr Ser Tyr Leu Glu Met Asp Leu Gln Cys Arg Asp Pro Asp Ile His
            260                 265                 270
Ala Ala Ala Phe Gly Thr Cys Leu Ala Ala Ser Val Ala Ala Pro Gly
        275                 280                 285
Ser Gly Arg Val Leu Tyr Ala Val Phe Ser Arg Asp Ser Arg Ser Ser
    290                 295                 300
Gly Gly Pro Gly Ala Gly Leu Cys Leu Phe Pro Leu Asp Lys Val His
305                 310                 315                 320
Ala Lys Met Glu Ala Asn Arg Asn Ala Cys Tyr Thr Gly Thr Arg Glu
                325                 330                 335
Ala Arg Asp Ile Phe Tyr Lys Pro Phe His Gly Asp Ile Gln Cys Gly
            340                 345                 350
Gly His Ala Pro Gly Ser Ser Lys Ser Phe Pro Cys Gly Ser Glu His
        355                 360                 365
Leu Pro Tyr Pro Leu Gly Ser Arg Asp Gly Leu Arg Gly Thr Ala Val
    370                 375                 380
Leu Gln Arg Gly Gly Leu Asn Leu Thr Ala Val Thr Val Ala Ala Glu
385                 390                 395                 400
Asn Asn His Thr Val Ala Phe Leu Gly Thr Ser Asp Gly Arg Ile Leu
                405                 410                 415
Lys Val Tyr Leu Thr Pro Asp Gly Thr Ser Ser Glu Tyr Asp Ser Ile
            420                 425                 430
Leu Val Glu Ile Asn Lys Arg Val Lys Arg Asp Leu Val Leu Ser Gly
        435                 440                 445
Asp Leu Gly Ser Leu Tyr Ala Met Thr Gln Asp Lys Val Phe Arg Leu
    450                 455                 460
Pro Val Gln Glu Cys Leu Ser Tyr Pro Thr Cys Thr Gln
465                 470                 475

<210> 15
<211> 254
<212> DNA
<213> Homo sapiens

<220>
<223> PLXNB2-206 cDNA (coding sequence)

<220>
<221> variation
<222> 1
<223> /replace="a,c,g,t"

<400> 15
nagacagatg tgaacttcca gggcaagaac ctggacaccg tgaaggtgtg gatgagtggt      60

ggaggtggcc gtgggaaacc cacagaggac gttcagaggc ttctgcagac acagggttcc 120

tccctgcacg tgggcagtga cttgctcaag ttcatggagc cggtgaccat gcaggaatct 180

gggaccttcg cctttcggac cccaaagctg tcccacgatg ccaacgagac gctgcccctg 240

cacctctacg tcaa 254

<210> 16
<211> 84
<212> PRT
<213> Homo sapiens

<220>
<223> PLXNB2-206 protein

<220>
<221> VARIANT
<222> 1
<223> Ala,Cys,Asp,Glu,Phe,Gly,His,Ile,Lys,Leu,Met,Asn,Pro,Gln,Arg,Ser,T
hr,Val,Trp,Tyr

<400> 16
Xaa Thr Asp Val Asn Phe Gln Gly Lys Asn Leu Asp Thr Val Lys Val
1               5                   10                  15
Trp Met Ser Gly Gly Gly Gly Arg Gly Lys Pro Thr Glu Asp Val Gln
            20                  25                  30
Arg Leu Leu Gln Thr Gln Gly Ser Ser Leu His Val Gly Ser Asp Leu
        35                  40                  45
Leu Lys Phe Met Glu Pro Val Thr Met Gln Glu Ser Gly Thr Phe Ala
    50                  55                  60
Phe Arg Thr Pro Lys Leu Ser His Asp Ala Asn Glu Thr Leu Pro Leu
65                  70                  75                  80
His Leu Tyr Val

<210> 17
<211> 483
<212> DNA
<213> Homo sapiens

<220>
<223> PLXNB2-213 cDNA (coding sequence)

<400> 17
ggacctgacg tcacagcggg agggccggtg gaagcgcgtc aacaccctta tgcactacaa 60

tgtccgggat ggagccaccc tcatcctgtc caaggtgggg gtctcccagc agccggagga 120

cagccagcag gacctgcctg gggagcgcca tgccctcctg gaggaggaga accgggtgtg 180

gcacctggtg cggccgaccg acgaggtgga cgagggcaag tccaagagag gcagcgtgaa 240

agagaaggag cggacgaagg ccatcaccga gatctacctg acgcggctgc tctcagtcaa 300

gggcacactg cagcagtttg tggacaactt cttccagagc gtgctggcct gggcacgcgg 360

tgccacctgc agtcaagtac ttcttcgact tcctggacga gcaggcagag aagcacaaca 420

tccaggatga agacaccatc cacatctgga agacgaacag cttaccgctc cggttctggg 480

tga 483

<210> 18
<211> 160
<212> PRT
<213> Homo sapiens

<220>
<223> PLXNB2-213 protein

<400> 18
Gly Pro Asp Val Thr Ala Gly Gly Pro Val Glu Ala Arg Gln His Pro
1               5                   10                  15
Tyr Ala Leu Gln Cys Pro Gly Trp Ser His Pro His Pro Val Gln Gly
            20                  25                  30
Gly Gly Leu Pro Ala Ala Gly Gly Gln Pro Ala Gly Pro Ala Trp Gly
        35                  40                  45
Ala Pro Cys Pro Pro Gly Gly Gly Glu Pro Gly Val Ala Pro Gly Ala
    50                  55                  60
Ala Asp Arg Arg Gly Gly Arg Gly Gln Val Gln Glu Arg Gln Arg Glu
65                  70                  75                  80
Arg Glu Gly Ala Asp Glu Gly His His Arg Asp Leu Pro Asp Ala Ala
                85                  90                  95
Ala Leu Ser Gln Gly His Thr Ala Ala Val Cys Gly Gln Leu Leu Pro
            100                 105                 110
Glu Arg Ala Gly Leu Gly Thr Arg Cys His Leu Gln Ser Ser Thr Ser
        115                 120                 125
Ser Thr Ser Trp Thr Ser Arg Gln Arg Ser Thr Thr Ser Arg Met Lys
    130                 135                 140
Thr Pro Ser Thr Ser Gly Arg Arg Thr Ala Tyr Arg Ser Gly Ser Gly
145                 150                 155                 160

<210> 19
<211> 1041
<212> DNA
<213> Homo sapiens

<220>
<223> PLXNB2-214 cDNA (coding sequence)

<400> 19
atgcacacgc tcttcctgga gctcctggag cagtacgtgg tggccaagaa ccccaagctg      60

atgctgcgca ggtctgagac tgtggtggag aggatgctgt ccaactggat gtccatctgc     120

ctgtaccagt acctcaagga cagtgccggg gagcccctgt acaagctctt caaggccatc     180

aaacatcagg tggaaaaggg cccggtggat gcggtacaga agaaggccaa gtacactctc     240

aacgacacgg ggctgctggg ggatgatgtg gagtacgcac ccctgacggt gagcgtgatc     300

gtgcaggacg agggagtgga cgccatcccg gtgaaggtcc tcaactgtga caccatctcc     360

caggtcaagg agaagatcat tgaccaggtg taccgtgggc agccctgctc ctgcggccca     420

ggccagacag cgtggtcctg gagtggcgtc cgggctccac agcgcagatc cgtcggacct     480

ggacctgacg tcacagcggg agggccggtg gaagcgcgtc aacaccctta tgcactacaa     540

tgtccgggat ggagccaccc tcatcctgtc caaggtgggg gtctcccagc agccggagga     600

cagccagcag gacctgcctg gagcgccatg cctcctggag gaggagaacc gggtgtggca 660

cctggtgcgg ccgaccgacg aggtggacga gggcaagtcc aagagaggca gcgtgaaaga 720

gaaggagcgg acgaaggcca tcaccgagat ctacctgacg cggctgctct cagtcaaggg 780

cacactgcag cagtttgtgg acaacttctt ccagagcgtg ctggcgcctg gcacgcggtg 840

ccacctgcag tcaagtactt cttcgacttc ctggacgagc aggcagagaa gcacaacatc 900

caggatgaag acaccatcca catctggaag acgaacagct taccgctccg gttctgggtg 960

aacatcctca agaaccccca cttcatcttt gacgtgcatg tcacgaggtg gtggacgcct 1020

cgctgtcagt catcgcgcag a 1041

<210> 20
<211> 347
<212> PRT
<213> Homo sapiens

<220>
<223> PLXNB2-214 protein

<400> 20

## Description

The present invention relates to a composition comprising an inhibitor of plexin-B1 and an inhibitor of plexin-B2, wherein the inhibitor of plexin-B1 and/or the inhibitor of plexin-B2 is/are preferably selected from (i) an inhibitor of the nucleic acid molecule encoding plexin-B1 and/or plexin-B2 selected from a small molecule, an aptamer, a siRNA, a shRNA, a miRNA, a morpholino, a ribozyme, an antisense nucleic acid molecule, a CRISPR-Cas9-based construct, a CRISPR-Cpf1-based construct, a meganuclease, a zinc finger nuclease, and a transcription activator-like (TAL) effector (TALE) nuclease, and/or (ii) an inhibitor of the plexin-B1 and/or plexin-B2 protein selected from a small molecule, an antibody or antibody mimetic, an aptamer, wherein the antibody mimetic is preferably selected from affibodies, adnectins, anticalins, DARPins, avimers, nanofitins, affilins, Kunitz domain peptides and Fynomers®.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Plexins are a family of transmembrane receptors for semaphorins, initially characterized in the context of axon guidance in the developing nervous system (Tamagnone 1990, Cell 99:71-80). Plexin-B1 has been shown to stably interact with ErbB-2 (Swiercz 2004, J Cell Biol 165:869-880). This interaction is critical for activation of the small GTPase RhoA by semaphorin ligands of Plexin-B1. The Rho family of small GTPases has been extensively studied for their role in invasion of cancer cells (Sahai 2002, Nat Rev Cancer 2: 133-42). RhoA and RhoC, in particular, are overexpressed in breast cancer and contribute to metastasis and poor outcome in breast cancer patients (Lin 2004, Breast Cancer Res Treat 84:49-60). Moreover, binding of the ligand Sema4D to its receptor plexin-B1 stimulates the kinase activity of ErbB-2 which leads to phosphorylation of plexin-B1 at two specific tyrosine residues (Swiercz 2009, Mol Cell Biol 29:6321-34). Plexin has been reported as a target protein for tumor diagnosis and therapy (US2010/119445).

Plexin-B1 and Plexin-B2 are highly homologous transmembrane receptors involved in cell-cell communication. WO 2012/107531 describes that an antagonist of plexin-B1 being capable of inhibiting the interaction between plexin-B1 and ErbB-2 can be used to treat metastatic cancer. WO 2012/135332 describes that the inhibition of plexin-B2-mediated angiogenin activity is useful for the treatment of cancer and the inhibition of angiogenesis. Moreover, EP2993185 reveals that an antibody blocking the interaction of plexin-B1 and semaphorin is useful to treat osteoporosis, multiple sclerosis and neoplastic diseases.

Although medical uses of plexins including B-type plexins are known there is an ongoing need for new treatment avenues for conditions or disease where treatment options are not available or not fully satisfactory. This need is addressed by the present application.

Accordingly the present invention relates in a first aspect to a composition comprising an inhibitor of plexin-B1 and an inhibitor of plexin-B2, wherein the inhibitor of plexin-B1 and/or the inhibitor of plexin-B2 is/are preferably selected from (i) an inhibitor of the nucleic acid molecule encoding plexin-B1 and/or plexin-B2 selected from a small molecule, an aptamer, a siRNA, a shRNA, a miRNA, a morpholino, a ribozyme, an antisense nucleic acid molecule, a CRISPR-Cas9-based construct, a CRISPR-Cpf1-based construct, a meganuclease, a zinc finger nuclease, and a transcription activator-like (TAL) effector (TALE) nuclease, and/or (ii) an inhibitor of the plexin-B1 and/or plexin-B2 protein selected from a small molecule, an antibody or antibody mimetic, an aptamer, wherein the antibody mimetic is preferably selected from affibodies, adnectins, anticalins, DARPins, avimers, nanofitins, affilins, Kunitz domain peptides and Fynomers®.

Plexins comprise a family of transmembrane receptors for semaphorins (Worzfeld & Offermanns, 2014). Based on homologies, plexins are divided into four subfamilies (A-D). The interaction of plexins with semaphorins is mediated by a highly conserved seven-blade β-propeller domain, the "sema domain" (Janssen et al., 2010; Liu et al., 2010; Nogi et al., 2010; Hota & Buck, 2012). The binding of semaphorins to plexins results in activation of a variety of signaling pathways (Jongbloets & Pasterkamp, 2014; Worzfeld & Offermanns, 2014; Verlinden et al. 2016). All plexins carry a GTPase-activating protein (GAP) domain in their intracellular part, which mediates the guanine nucleotide exchange of R-Ras, M-Ras and Rap1. The inhibition of R-Ras has been linked to the inhibition of the signal transduction of integrins. Plexins of the B-subfamily have a PDZ binding motif at their C-terminus, which mediates a stable interaction with the Rho guanine nucleotide exchange factors (RhoGEFs) LARG and PDZ-RhoGEF. An activation of plexins thereby leads to an activation of the small GTPases RhoA and RhoC (Worzfeld & Offermanns, 2014).

Plexin-B1 and Plexin-B2 share a high sequence homology. Both receptors are activated by the semaphorins 4A and 4D and carry out redundant functions in many tissues. Inhibition of Plexin-B1 can often be compensated by Plexin-B2 and *vice versa* (Perälä et al., 2011; Xia et al., 2015; Daviaud et al., 2016).

As used herein "plexin-B1" (also known as PLXNB1, PLEXIN 5, PLXN5, TRANSMEMBRANE PROTEIN SEP or SEP) refers to the B1 member of the plexin family. Plexin-B1 is preferably human plexin-B1. The cDNA sequence of human plexin-B1 is shown in SEQ ID NO: 1 and the amino acid sequence in SEQ ID NO: 3. Also a shorter variant of plexin-B1 can be found in the ensemble genome browser (Human GRCh38.p12). The cDNA sequence of the human plexin-B1 variant is shown in SEQ ID NO: 5 and the amino acid sequence in SEQ ID NO: 6. Hence, the human plexin-B1 has preferably a cDNA sequence of SEQ ID NO: 1 or 5 and an amino acid sequence of SEQ ID NO: 3 or 6. Among these sequences SEQ ID NOs 1 and 3 are preferred.

As used herein "plexin-B2" (also known as PLXNB2) refers to the B2 member of the plexin family. In accordance with the invention, plexin-B2 is preferably human plexin-B2. Also in accordance with the invention, the inhibitor of plexin-B1 is preferably an inhibitor of human plexin-B1 and the inhibitor of plexin-B2 is preferably an inhibitor human plexin-B2. The cDNA sequence of human plexin-B2 is shown in SEQ ID NO: 2 and the amino acid sequence in SEQ ID NO: 4. Seven shorter variants of plexin-B2 can be found in the ensemble genome browser (Human GRCh38.p12). The cDNA sequences of the human plexin-B2 variant are shown in SEQ ID NOs 7, 9, 11, 13, 15, 17 and 19, and the amino acid sequences in SEQ ID NOs 8, 10, 12, 14, 16, 18 and 20. Hence, the human plexin-B2 has preferably a cDNA sequence of SEQ ID NOs 2, 7, 9, 11, 13, 15, 17 or 19 and an amino acid sequence of SEQ ID NOs 4, 8, 10, 12, 14, 16, 18 or 20. Among these sequences SEQ ID NOs 2 and 4 are preferred.

The term "nucleic acid sequence", "nucleic acid sequence molecule" or "nucleotide sequence", in accordance with the present invention, includes DNA and RNA. The cDNA sequences of above SEQ ID NOs, wherein T is replaced by U are the mRNA sequences.

The inhibitor is also referred to herein below as a compound. Based on the activity and target of the compound, the compound is described as a compound inhibiting the expression of the nucleic acid molecule encoding plexin-B1 and/or plexin-B2 or as a compound inhibiting the protein plexin-B1 and/or plexin-B2. The nucleic acid molecule encoding plexin-B1 and/or plexin-B2 is also referred to herein below as nucleic acid molecule according to the invention and the protein plexin-B1 and/or plexin-B2 as protein according to the invention. Also with respect to the nucleic acid sequences, the amino acid sequences preferably correspond to both human plexin-B1 and human plexin-B2.

As will be further detailed herein below, the inhibitor of plexin-B1 and the inhibitor of plexin-B2 may be one compound or two distinct compounds. In the first case the compound is either bi-specific for plexin-B1 and plexin-B2 or is specific for both plexin-B1 and plexin-B2. In the latter case the two distinct compounds are preferably both an inhibitor of a nucleic acid molecule according to the invention or are both an inhibitor of the protein according to the invention. More preferably both compounds belong to the same class of compounds as described herein below, such as two siRNAs or two antibodies, wherein each is specific for plexin-B1 and plexin-B2, respectively.

A compound inhibiting the expression of the nucleic acid molecule and/or the protein according to the invention is in accordance with the present invention (i) is a compound lowering or preventing the transcription of the gene encoding the nucleic acid molecule and/or the protein according to the invention, or (ii) is a compound lowering or preventing the translation of the mRNA encoding the the protein according to the invention. Compounds of (i) include compounds interfering with the transcriptional machinery and/or its interaction with the promoter of said gene and/or with expression control elements remote from the promoter such as enhancers. Compounds of (ii) include compounds interfering with the translational machinery.

The compound inhibiting the expression of the nucleic acid molecule and/or the protein according to the invention specifically inhibits the expression of the nucleic acid molecule encoding plexin-B1 and/or plexin-B2 protein, and/or the plexin-B1 and/or plexin-B2 protein. With respect to the inhibitor of the plexin-B1 and/or plexin-B2 protein it is preferred that the inhibitor specifically binds to the extracellular domain of plexin-B1 and/or plexin-B2 protein. The extracellular domain of human plexin-B1 corresponds to amino acid positions 20 to 1490 of SEQ ID NO: 3. The extracellular domain of human plexin-B2 corresponds to amino acid positions 20 to 1197of SEQ ID NO: 4. Binding to the extracellular domain avoids the necessity of an intracellular delivery of such an inhibitor.

Preferably, the transcription of the nucleic acid molecule and/or the protein according to the invention or the translation of the protein according to the invention is reduced by at least 50%, more preferred at least 75% such as at least 90% or 95%, even more preferred at least 98% and most preferred by about 100% (e.g., as compared to the same experimental set up in the absence of the compound).

A compound inhibiting the activity of the nucleic acid molecule and/or the protein according to the invention in accordance with the present invention causes said nucleic acid molecule and/or protein to perform its/their function with lowered efficiency. The compound inhibiting the activity of the nucleic acid molecule and/or the protein according to the invention specifically inhibits the activity of said nucleic acid molecule and/or protein. The compound inhibiting the activity of the nucleic acid molecule and/or the protein according to the invention may specifically inhibit the activity of said nucleic acid molecule and/or protein by interacting with the nucleic acid molecule and/or protein itself or by specifically inhibiting cells that produce said nucleic acid molecule and/or produce said protein and/or bind to said protein. Preferably, the activity of the nucleic acid molecule and/or the protein according to the invention is reduced by at least 50%, more preferred at least 75% such as at least 90% or 95%, even more preferred at least 98%, and most preferably about 100% (e.g., as compared to the same experimental set up in the absence of the compound).

The activity of the nucleic acid molecule and/or the protein according to the invention is in accordance with this invention its/their capability to induce tumorigenesis, in particular of colon cancer or to induce bone formation from osteoblasts, in particular in osteoporosis (see also the appended examples).

The efficiency of inhibition by an inhibitor can be quantified by methods comparing the level of activity in the presence of the inhibitor to that in the absence of the inhibitor. For example, the change in the amount of the nucleic acid molecule and/or the protein according to the invention formed may be used in the measurement. The efficiency of several inhibitors may be determined simultaneously in high-throughput formats. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within a short time, mixtures of, for example 10, 20, 30, 40, 50 or 100 test compounds may be added to each well. In case a well exhibits the expected activity, said mixture of test compounds may be de-convoluted to identify the one or more test compounds in said mixture giving rise to said activity.

For intracellular delivery the compounds inhibiting the expression and/or the activity of the nucleic acid molecule and/or the protein according to the invention may be formulated as vesicles, such as liposomes or exosomes. Liposomes have attracted great interest because of their specificity and the duration of action they offer from the standpoint of drug delivery. Liposomal cell-type delivery systems have been used to effectively deliver nucleic acids, such as siRNA in vivo into cells (Zimmermann et al. (2006) Nature, 441:111-114). Liposomes are unilamellar or multilamellar vesicles which have a membrane formed from a lipophilic material and an aqueous interior. The aqueous portion contains the composition to be delivered. Cationic liposomes possess the advantage of being able to fuse to the cell wall. Non-cationic liposomes, although not able to fuse as efficiently with the cell wall, are phagocytosed by macrophages and other cells in vivo. Exosomes are lipid packages which can carry a variety of different molecules including RNA (Alexander et al. (2015), Nat Commun; 6:7321). The exosomes including the molecules comprised therein can be taken up by recipient cells. Hence, exosomes are important mediators of intercellular communication and regulators of the cellular niche. Exosomes are useful for diagnostic and therapeutic purposes, since they can be used as delivery vehicles, e.g. for contrast agents or drugs.

The "small molecule" as used herein is preferably an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. The organic molecule is preferably an aromatic molecule and more preferably a heteroaromatic molecule. In organic chemistry, the term aromaticity is used to describe a cyclic (ring-shaped), planar (flat) molecule with a ring of resonance bonds that exhibits more stability than other geometric or connective arrangements with the same set of atoms. Aromatic molecules are very stable, and do not break apart easily to react with other substances. In a heteroaromatic molecule at least one of the atoms in the aromatic ring is an atom other than carbon, e.g. N, S, or O. For all above-described organic molecules the molecular weight is preferably in the range of 200 Da to 1500 Da and more preferably in the range of 300 Da to 1000 Da.

Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 Da, or less than about 1000 Da such as less than about 500 Da, and even more preferably less than about 250 Da. The size of a small molecule can be determined by methods well-known in the art, e.g., mass spectrometry. The small molecules may be designed, for example, based on the crystal structure of the target molecule, where sites presumably responsible for the biological activity can be identified and verified in in vivo assays such as in vivo high-throughput screening (HTS) assays.

The term "antibody" as used in accordance with the present invention comprises, for example, polyclonal or monoclonal antibodies. Furthermore, also derivatives or fragments thereof, which still retain the binding specificity to the target, e.g. the plexin-B1 or plexin-B2 of SEQ ID NO: 3 or 4, are comprised in the term "antibody". Antibody fragments or derivatives comprise, inter alia, Fab or Fab' fragments, Fd, F(ab')₂, Fv or scFv fragments, single domain VH or V-like domains, such as VhH or V-NAR-domains, as well as multimeric formats such as minibodies, diabodies, tribodies or triplebodies, tetrabodies or chemically conjugated Fab'-multimers (see, for example, Harlow and Lane "Antibodies, A Laboratory Manual", Cold Spring Harbor Laboratory Press, 1988; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. The first antigen can be found on the protein according to the invention. The second antigen may, for example, be a tumor marker that is specifically expressed on cancer cells or a certain type of cancer cells. Non-limiting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847). Further details on bispecific antibodies will provided herein below.

The term "antibody" also includes embodiments such as chimeric (human constant domain, non-human variable domain), single chain and humanised (human antibody with the exception of non-human CDRs) antibodies.

Various techniques for the production of antibodies are well known in the art and described, e.g. in Harlow and Lane (1988) and (1999) and Altshuler et al., 2010, loc. cit. Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Köhler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared de novo using various display methods such as phage, ribosomal, mRNA, or cell display. A suitable system for the expression of the recombinant (humanised) antibodies may be selected from, for example, bacteria, yeast, insects, mammalian cell lines or transgenic animals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for an epitope of plexin-B1 and/or plexin-B2. Surface plasmon resonance as employed in the BIAcore system can be used to increase the efficiency of phage antibodies.

Antibodies against plexin-B1 (e.g. WO 2012/135332 and EP2993185) and plexin-B2 (e.g. WO 2012/107531) are known in the art.

As used herein, the term "antibody mimetics" refers to compounds which, like antibodies, can specifically bind antigens, such as the plexin-B1 and/or plexin-B2 of SEQ ID NO: 2 or 4 in the present case, but which are not structurally related to antibodies. Antibody mimetics are usually artificial peptides or proteins with a molar mass of about 3 to 20 kDa. For example, an antibody mimetic may be selected from the group consisting of affibodies, adnectins, anticalins, DARPins, avimers, nanofitins, affilins, Kunitz domain peptides and Fynomers®.These polypeptides are well known in the art and are described in further detail herein below.

The term "affibody", as used herein, refers to a family of antibody mimetics which is derived from the Z-domain of staphylococcal protein A. Structurally, affibody molecules are based on a three-helix bundle domain which can also be incorporated into fusion proteins. In itself, an affibody has a molecular mass of around 6kDa and is stable at high temperatures and under acidic or alkaline conditions. Target specificity is obtained by randomisation of 13 amino acids located in two alpha-helices involved in the binding activity of the parent protein domain (Feldwisch J, Tolmachev V.; (2012) Methods Mol Biol. 899:103-26).

The term "adnectin" (also referred to as "monobody"), as used herein, relates to a molecule based on the 10th extracellular domain of human fibronectin III (10Fn3), which adopts an Ig-like β-sandwich fold of 94 residues with 2 to 3 exposed loops, but lacks the central disulphide bridge (Gebauer and Skerra (2009) Curr Opinion in Chemical Biology 13:245-255). Adnectins with the desired target specificity, i.e. against plexin-B1 and/or plexin-B2, can be genetically engineered by introducing modifications in specific loops of the protein.

The term "anticalin", as used herein, refers to an engineered protein derived from a lipocalin (Beste G, Schmidt FS, Stibora T, Skerra A. (1999) Proc Natl Acad Sci U S A. 96(5):1898-903; Gebauer and Skerra (2009) Curr Opinion in Chemical Biology 13:245-255). Anticalins possess an eight-stranded - barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

As used herein, the term "DARPin" refers to a designed ankyrin repeat domain (166 residues), which provides a rigid interface arising from typically three repeated β-turns. DARPins usually carry three repeats corresponding to an artificial consensus sequence, wherein six positions per repeat are randomised. Consequently, DARPins lack structural flexibility (Gebauer and Skerra, 2009).

The term "avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with the desired binding specificity, i.e. for plexin-B1 and/or plexin-B2, can be selected, for example, by phage display techniques. The binding specificity of the different A-domains contained in an avimer may, but does not have to be identical (Weidle UH, et al., (2013), Cancer Genomics Proteomics; 10(4):155-68).

A "nanofitin" (also known as affitin) is an antibody mimetic protein that is derived from the DNA binding protein Sac7d of Sulfolobus acidocaldarius. Nanofitins usually have a molecular weight of around 7kDa and are designed to specifically bind a target molecule, such as e.g. plexin-B1 and/or plexin-B2, by randomising the amino acids on the binding surface (Mouratou B, Béhar G, Paillard-Laurance L, Colinet S, Pecorari F., (2012) Methods Mol Biol.; 805:315-31).

The term "affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity, i.e. against plexin-B1 and/or plexin-B2, is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDA and domains with the required target specificity, i.e. against plexin-B1 and/or plexin-B2, can be selected by display techniques such as phage display (Weidle et al., (2013), Cancer Genomics Proteomics; 10(4):155-68).

As used herein, the term "Fynomer®" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

Nucleic acid aptamers are nucleic acid species that normally consist of (usually short) strands of oligonucleotides. Typically, they have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers are usually peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site the two cysteins lateral chains thereof being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminatory recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamers' inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The small molecule, antibody or antibody mimetic and aptamer can also be generated in the format of drug-conjugates. In this case the small molecule, antibody or antibody mimetic and aptamer in itself may not have an inhibitory effect but the inhibitory effect is only conferred by the drug. The small molecule, antibody or antibody mimetic and aptamer confer the site-specificity binding of the drug to cells producing and/or binding to the protein according to the invention. The drug is preferably capable to kill cells producing and/or binding to the protein according to the invention. Hence, by combining the targeting capabilities of moelcules binding to the protein according to the invention with the cell-killing ability of the drug, the drug conjugates become inhibitors that allow for discrimination between healthy and diseased tissue and cells. Cleavable and non-cleavable linkers to design drug conjugates are known in the art. Non-limiting examples of drugs being capable of killing cells are cytostaic drugs and radioisotopes that deliver radiation directly to the cancer cells.

It is furthermore possible to confine the binding and/or inhibitory activity of the small molecule, antibody or antibody mimetic and aptamer to certain tissues or cell-types, in particular diseased tissues or cell-types. For instance, probodies may be designed. In a probody the small molecule, antibody or antibody mimetic or aptamer is bound to a masking peptide which limits or prevents binding to the protein according to the invention and which masking peptide can be cleaved by a protease. Proteases are enzymes that digest proteins into smaller pieces by cleaving specific amino acid sequences known as substrates. In normal healthy tissue, protease activity is tightly controlled. In cancer cells, protease activity is upregulated. In healthy tissue or cells, where protease activity is regulated and minimal, the target-binding region of the probody remains masked and is thus unable to bind. On the other hand, in diseased tissue or cells, where protease activity is upregulated, the target-binding region of the probody gets unmasked and is thus able to bind and/or inhibit.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene for which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1 to 5 nucleotides, more preferably from 1 to 3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A preferred example of a siRNA is an Endoribonuclease-prepared siRNA (esiRNA). An esiRNA is a mixture of siRNA oligos resulting from cleavage of a long double-stranded RNA (dsRNA) with an endoribonuclease such as *Escherichia coli* RNase III or dicer. esiRNAs are an alternative concept to the usage of chemically synthesized siRNA for RNA Interference (RNAi). For the generation of esiRNAs a cDNA of a mRNA template may be amplified by PCR and tagged with two bacteriophage-promotor sequences. RNA polymerase is then used to generate long double stranded RNA that is complentary to the target-gene cDNA. This complentary RNA may be subsequently digested with RNase III from *Escherichia coli* to generate short overlapping fragments of siRNAs with a length between 18-25 base pairs. This complex mixture of short double stranded RNAs is similar to the mixture generated by dicer cleavage in vivo and is therefore called endoribonuclease-prepared siRNA or short esiRNA. Hence, esiRNA are a heterogeneous mixture of siRNAs that all target the same mRNA sequence. esiRNAs lead to highly specific and effective gene silencing.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules. Endogenously present miRNA molecules regulate gene expression by binding to a complementary mRNA transcript and triggering of the degradation of said mRNA transcript through a process similar to RNA interference. Accordingly, exogenous miRNA may be employed as an inhibitor of plexin-B1 and/or plexin-B2 after introduction into the respective cells.

Morpholinos (or morpholino oligonucleotides) are synthetic nucleic acid molecules having a length of about 20 to 30 nucleotides and, typically about 25 nucleotides. Morpholinos bind to complementary sequences of target transcripts by standard nucleic acid base-pairing. They have standard nucleic acid bases which are bound to morpholine rings instead of deoxyribose rings and linked through phosphorodiamidate groups instead of phosphates (see, e.g., Summerton 1997, Antisense & Nucleic Acid Drug Development 7 (3): 187-95). Due to replacement of anionic phosphates into the uncharged phosphorodiamidate groups, ionization in the usual physiological pH range is prevented, so that morpholinos in organisms or cells are uncharged molecules. The entire backbone of a morpholino is made from these modified subunits. Unlike inhibitory small RNA molecules, morpholinos do not degrade their target RNA molecules. Rather, they sterically block binding to a target sequence within a RNA and simply getting in the way of molecules that might otherwise interact with the RNA (see, e.g., Summerton 1999, Biochimica et Biophysica Acta 1489 (1): 141-58).

A ribozyme (from ribonucleic acid enzyme, also called RNA enzyme or catalytic RNA) is an RNA molecule that catalyses a chemical reaction. Many natural ribozymes catalyse either their own cleavage or the cleavage of other RNAs, but they have also been found to catalyse the aminotransferase activity of the ribosome. Non-limiting examples of well-characterised small self-cleaving RNAs are the hammerhead, hairpin, hepatitis delta virus, and *in vitro*-selected lead-dependent ribozymes, whereas the group I intron is an example for larger ribozymes. The principle of catalytic self-cleavage has become well established in recent years. The hammerhead ribozymes are characterised best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: A region of interest of the RNA, which contains the GUC (or CUC) triplet, is selected. Two oligonucleotide strands, each usually with 6 to 8 nucleotides, are taken and the catalytic hammerhead sequence is inserted between them. The best results are usually obtained with short ribozymes and target sequences.

A recent development, also useful in accordance with the present invention, is the combination of an aptamer, recognizing a small compound, with a hammerhead ribozyme. The conformational change induced in the aptamer upon binding the target molecule can regulate the catalytic function of the ribozyme.

The term "antisense nucleic acid molecule", as used herein, refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

The antisense oligonucleotide is preferably a LNA-GapmeR, an Antagomir, or an antimiR.

LNA-GapmeRs or simply GapmeRs are potent antisense oligonucleotides used for highly efficient inhibition of mRNA function. GapmeRs function by RNase H dependent degradation of complementary RNA targets. They are an excellent alternative to siRNA for knockdown of mRNA. They are advantageously taken up by cell without transfection reagents. GapmeRs contain a central stretch of DNA monomers flanked by blocks of LNAs. The GapmeRs are preferably 14-16 nucleotides in length and are optionally fully phosphorothioated. The DNA gap activates the RNAse H-mediated degradation of targeted RNAs and is also suitable to target transcripts directly in the nucleus. The LNA-GapmeR technology is well established. LNA-GapmeRs are routinely designed using established algorithms. LNA-GapmeRs to a selected target are commercially available including positive and negative controls, for example, from Exiqon.

AntimiRs are oligonucleotide inhibitors that were initially designed to be complementary to a miRNA. AntimiRs against miRNAs have been used extensively as tools to gain understanding of specific miRNA functions and as potential therapeutics. As used herein, the AntimiRs are designed to be complementary to mRNA of plexin-B1 and/or plexin-B2. AntimiRs are preferably 14 to 23 nucleotides in length.

AntimiRs are preferably AntagomiRs. AntagomiRs are synthetic 2-O-methyl RNA oligonucleotides, preferably of 21 to 23 nucleotides which are preferably fully complementary to the selected target RNA. While AntagomiRs were initially designed against miRNAs they may also be designed against mRNAs. AntagomiRs are preferably synthesized with 2'-OMe modified bases (2'-hydroxyl of the ribose is replaced with a methoxy group), phosphorothioate (phosphodiester linkages are changed to phosphorothioates) on the first two and last four bases, and an addition of cholesterol motif at 3' end through a hydroxyprolinol modified linkage. The addition of 2'-OMe and phosphorothioate modifications improve the bio-stability whereas cholesterol conjugation enhances distribution and cell permeation of the AntagomiRs.

Antisense molecules (including antisense oligonucleotides, such as LNA-GapmeR, an Antagomir, an antimiR), siRNAs and shRNAs of the present invention are preferably chemically synthesized using a conventional nucleic acid synthesizer. Suppliers of nucleic acid sequence synthesis reagents include Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK).

The ability of antisense molecules (including antisense oligonucleotides, such as LNA-GapmeR, an Antagomir, an antimiR), siRNA, and shRNA to potently, but reversibly, silence or inhibit a mRNA *in vivo* makes these molecules particularly well suited for use in the pharmaceutical composition and medical uses of the invention. Ways of administering siRNA to humans are described in De Fougerolles et al., Current Opinion in Pharmacology, 2008, 8:280-285. Such ways are also suitable for administering other small RNA molecules like antisense oligonucleotides or shRNAs. Accordingly, such pharmaceutical compositions may be administered directly formulated as a saline, via liposome based and polymer-based nanoparticle approaches, as conjugated or complexation pharmaceutical compositions, or via viral delivery systems. Direct administration comprises injection into tissue, intranasal and intratracheal administration. Liposome based and polymer-based nanoparticle approaches comprise the cationic lipid Genzyme Lipid (GL) 67, cationic liposomes, chitosan nanoparticles and cationic cell penetrating peptides (CPPs). Conjugated or complexation pharmaceutical compositions comprise PEI-complexed antisense molecules (including antisense oligonucleotides), siRNA, or shRNA. Further, viral delivery systems comprise influenza virus envelopes and virosomes.

The antisense molecules (including antisense oligonucleotides, such as LNA-GapmeR, an Antagomir, an antimiR), siRNAs, shRNAs may comprise modified nucleotides such as locked nucleic acids (LNAs). The ribose moiety of an LNA nucleotide is modified with an extra bridge connecting the 2' oxygen and 4' carbon. The bridge "locks" the ribose in the 3'*-endo* (North) conformation, which is often found in the A-form duplexes. LNA nucleotides can be mixed with DNA or RNA residues in the oligonucleotide whenever desired. Such oligomers are synthesized chemically and are commercially available. The locked ribose conformation enhances base stacking and backbone pre-organization. This significantly increases the hybridization properties (melting temperature) of oligonucleotides.

CRISPR/Cas9, as well as CRISPR-Cpf1, technologies are applicable in nearly all cells/model organisms and can be used for knock out mutations, chromosomal deletions, editing of DNA sequences and regulation of gene expression. The regulation of the gene expression can be manipulated by the use of a catalytically dead Cas9 enzyme (dCas9) that is conjugated with a transcriptional repressor to repress transcription a specific gene, here the plexin-B1 and/or plexin-B2 gene. Similarly, catalytically inactive, "dead" Cpf1 nuclease (CRISPR from Prevotella and Francisella-1) can be fused to synthetic transcriptional repressors or activators to downregulate endogenous promoters, e.g. the promoter which controls plexin-B1 and/or plexin-B2 expression. Alternatively, the DNA-binding domain of zinc finger nucleases (ZFNs) or transcription activator-like effector nucleases (TALENs) can be designed to specifically recognize the plexin-B1 and/or plexin-B2 gene or its promoter region or its 5'-UTR thereby inhibiting the expression of the plexin-B1 and/or plexin-B2 gene.

Inhibitors provided as inhibiting nucleic acid molecules that target the plexin-B1 and/or plexin-B2 gene or a regulatory molecule involved in plexin-B1 and/or plexin-B2 expression are also envisaged herein. Such molecules, which reduce or abolish the expression of plexin-B1 and/or plexin-B2 or a regulatory molecule include, without being limiting, meganucleases, zinc finger nucleases and transcription activator-like (TAL) effector (TALE) nucleases. Such methods are described in Silva et al., Curr Gene Ther. 2011;11(1):11-27; Miller et al., Nature biotechnology. 2011;29(2):143-148, and Klug, Annual review of biochemistry. 2010; 79:213-231.

Herein above a number of examples of nucleotide-based inhibitors were described, in particular a siRNA, a shRNA, and an antisense nucleic acid molecule. It is preferred that these nucleotide-based inhibitors and other nucleotide-based inhibitors of plexin-B1 and/or plexin-B2 comprise (a) a nucleic acid sequence which comprises or consists of a nucleic acid sequence being complementary to at least 12 continuous nucleotides of a nucleic acid sequence selected from SEQ ID NOs 1, 2, 5, 7, 9, 11, 13, 15, 17 and 19, preferably SEQ ID NOs 1 and 2 (or both), (b) a nucleic acid sequence which comprises or consists of a nucleic acid sequence which is at least 70% identical to the complementary strand of one or more nucleic acid sequences selected from SEQ ID NOs 1, 2, 5, 7, 9, 11, 13, 15, 17 and 19, preferably SEQ ID NOs 1 and 2, (c) a nucleic acid sequence which comprises or consists of a nucleic acid sequence according to (a) or (b), wherein the nucleic acid sequence is DNA or RNA, (d) an expression vector expressing the nucleic acid sequence as defined in any one of (a) to (c), preferably under the control of a cancer-specific promoter and/or a bone-specific promoter, or (e) a host comprising the expression vector of (d).

The nucleic acid sequence according to item (a) of this further preferred embodiment of the invention comprises or consists of a sequence which is with increasing preference complementary to at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides of one or more selected from SEQ ID NOs 1, 2, 5, 7, 9, 11, 13, 15, 17 and 19, preferably SEQ ID NOs 1 and 2 (or both). The nucleic acid sequence according to item (a) comprises or consists of antisense an oligonucleotide. Hence, the nucleic acid sequence according to item (a) reflects the above-mentioned basic principle of the antisense technology which is the use of an oligonucleotide for silencing a selected target RNA through the exquisite specificity of complementary-based pairing. Therefore, it is to be understood that the nucleic acid sequence according to item (a) is preferably in the format of a siRNA, shRNA or an antisense oligonucleotide as defined herein above. The antisense oligonucleotides are preferably LNA-GapmeRs, AntagomiRs, or antimiRs as defined herein above.

The nucleic acid sequence according to item (b) requiring at least 70% identity to the complementary strand of one or more nucleic acid sequences selected from SEQ ID NOs 1, 2, 5, 7, 9, 11, 13, 15, 17 and 19, preferably SEQ ID NOs 1 and 2 is considerably longer than the nucleic acid sequence according to item (a) which comprises an antisense oligonucleotide and comprises at least 12 continuous nucleotides of a nucleic acid sequence selected from SEQ ID NOs 1, 2, 5, 7, 9, 11, 13, 15, 17 and 19, preferably SEQ ID NOs 1 or 2 (or both). A nucleic acid sequence according to item (b) of the above preferred embodiment of the invention is capable of interacting with, more specifically hybridizing with the target plexin-B1 of plexin-B2. By formation of the hybrid the function of the respective plexin is reduced or blocked.

The sequence identity of the molecule according to item (b) in connection with a sequence selected from SEQ ID NOs 1, 2, 5, 7, 9, 11, 13, 15, 17 and 19, preferably SEQ ID NOs 1 and 2 is with increasing preference at least 75%, at least 80%, at least 85%, at least 90%, at least 92.5%, at least 95%, at least 98%, at least 99% and 100%. Preferably, the BLAST (Basic Local Alignment Search Tool) program is used for determining the sequence identity with regard to SEQ ID NOs 1, 2, 5, 7, 9, 11, 13, 15, 17 and 19, preferably SEQ ID NO: 1 and/or 2.

In the nucleic acid sequence according to item (c) the nucleotide sequences may be RNA or DNA. RNA or DNA encompasses chemically modified RNA nucleotides or DNA nucleotides. As commonly known RNA comprises the nucleotide U while DNA comprises the nucleotide T.

In accordance with items (d) and (e) of the above preferred embodiment the inhibitor may also be an expression vector or host, respectively being capable of producing a nucleic acid sequence as defined in any one of items (a) to (c).

An expression vector may be a plasmid that is used to introduce a specific transcript into a target cell. Once the expression vector is inside the cell, the protein that is encoded by the gene is produced by the cellular-transcription and translation machinery ribosomal complexes. The plasmid is in general engineered to contain regulatory sequences that act as enhancer and/or promoter regions and lead to efficient transcription of the transcript. In accordance with the present invention the expression vector preferably contains a cancer-specific promoter or and/or bone-specific promoter. Such promoter may allow a more targeted treatment of the disease site of the disease described herein below, in particular cancer or bone diseases.

Non-limiting examples of expression vectors include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), and pXT1 (Stratage). Examples for plasmid vectors suitable for Pichia pastoris comprise e.g. the plasmids pAO815, pPIC9K and pPIC3.5K (all Intvitrogen). For the formulation of a pharmaceutical composition as described herein below a suitable vector is selected in accordance with good manufacturing practice. Such vectors are known in the art, for example, from Ausubel et al, Hum Gene Ther. 2011 Apr; 22(4):489-97 or Allay et al., Hum Gene Ther. May 2011; 22(5): 595-604.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside* ("IPTG"). For recombinant expression and secretion, the polynucleotide of interest may be ligated between e.g. the PelB leader signal, which directs the recombinant protein in the periplasm and the gene III in a phagemid called pHEN4 (described in Ghahroudi et al, 1997, FEBS Letters 414:521-526). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991, Biochem J. 227:277-279; Bebbington et al. 1992, Bio/Technology 10:169-175). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. For vector modification techniques, see Sambrook and Russel (2001), Molecular Cloning: A Laboratory Manual, 3 Vol. Generally, vectors can contain one or more origins of replication (ori) and inheritance systems for cloning or expression, one or more markers for selection in the host, e.g., antibiotic resistance, and one or more expression cassettes. Suitable origins of replication (ori) include, for example, the Col E1, the SV40 viral and the M 13 origins of replication.

The sequences to be inserted into the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e.g., translation initiation codon, promoters, enhancers, and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers, and/or naturally-associated or heterologous promoter regions. Preferably, the nucleotide sequence as defined in item (a) of the above preferred embodiment of the invention is operatively linked to such expression control sequences allowing expression in prokaryotic or eukaryotic cells.

The host may be a prokaryotic or eukaryotic cell. A suitable eukaryotic host may be a mammalian cell, an amphibian cell, a fish cell, an insect cell, a fungal cell or a plant cell. Representative examples of bacterial cells are *E*. *coli*, *Streptomyces* and *Salmonella typhimurium* cells; of fungal cells are yeast cells; and of insect cells are *Drosophila* S2 and *Spodoptera* Sf9 cells. It is preferred that the cell is a mammalian cell such as a human cell. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. The cell may be a part of a cell line, preferably a human cell line. Appropriate culture mediums and conditions for the above-described host cells are known in the art. The host is preferably a host cell and more preferably an isolated host cell. The host is also preferably a non-human host.

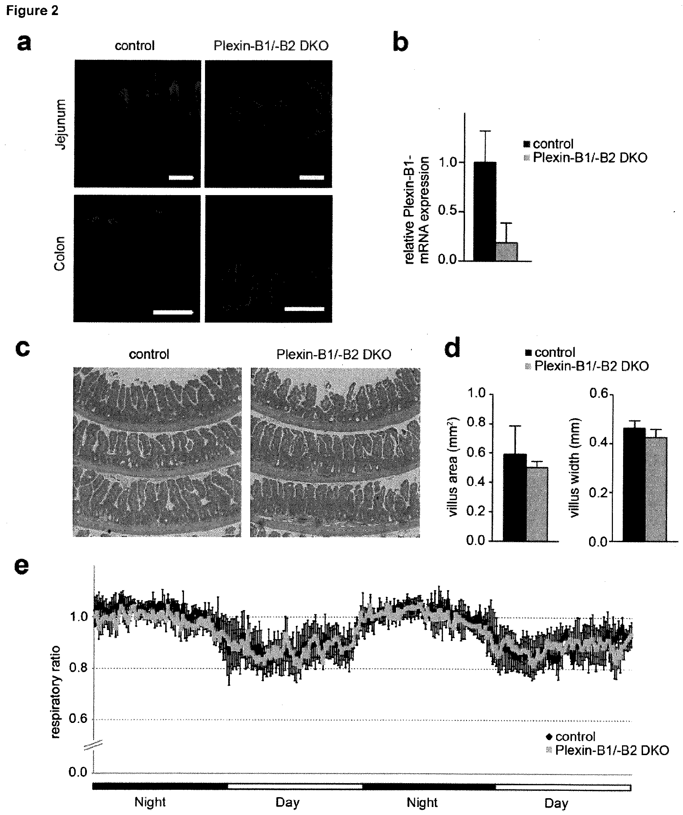

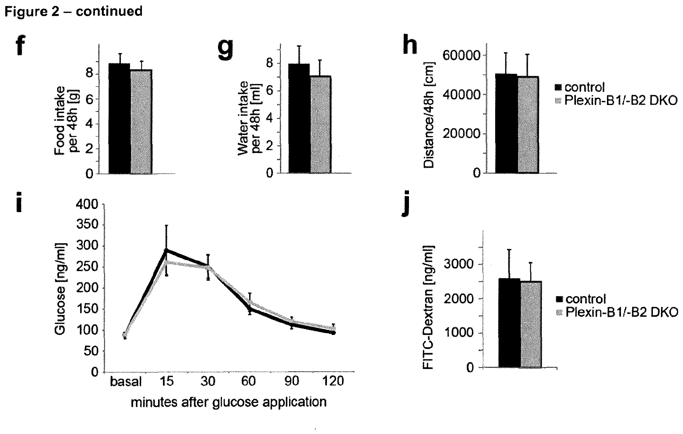

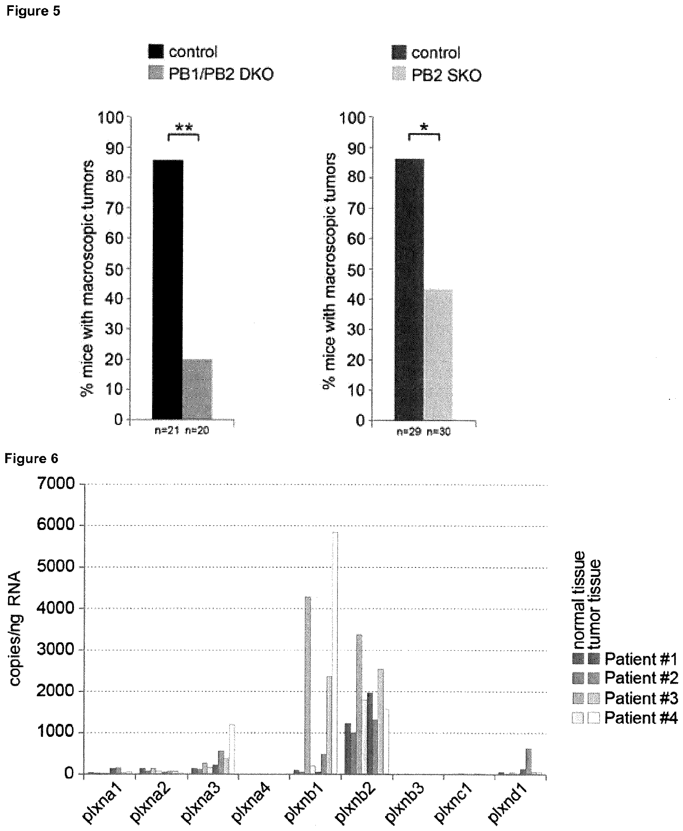

As is shown herein below in Example 1, under physiological conditions an inhibition of plexin-B1 and plexin-B2 function in the normal intestinal epithelium is compensated and stays functionally irrelevant (Fig. 2). By contrast, in tumors a dual inhibition of the function of plexin-B1 and plexin-B2 leads to a significant reduction in tumor formation (Fig. 5) and in particular a reduction which is superior as compared to the inhibition of plexin-B1 or plexin-B2. Hence, surprisingly a specific effect on tumor cells was found whereas the normal cells surrounding the tumor stayed unaffected. It was in particular unexpected that the normal cells were able to compensate the double inhibition of plexin-B1 and plexin-B2 whereas the tumor cells were not. While this is demonstrated for colon cancer, it is believed that also other cancer types can be treated in a similar manner by the double inhibition of plexin-B1 and plexin-B2.

As is furthermore shown herein below in Example 2, the inhibition of plexin-B1 and plexin-B2 advantageously promotes bone formation. Based on the data in Example 2, it can be concluded that only the double inhibition of both plexin-B1 and plexin-B2 is sufficient in order to overcome the inhibitory effect that plexin-B1 and plexin-B2 separately from each other have on the differentiation of osteoblasts into bone. The inhibition of plexin-B1 and plexin-B2 is therefore a *bona fide* novel treatment option for bone diseases and in particular osteoporosis. The inhibition of plexin-B1 and plexin-B2 is particularly advantageous since it not merely stops bone loss - as most currently available treatment options - but promotes bone formation. The progression of the bone disease may not only be stopped or slowed down, but an amelioration of the bone disease is expected to be achieved.

Thus, it is shown herein that the dual inhibition of plexin-B1 and plexin-B2 has surprising beneficial effects in the treatment of diseases. Therefore, the composition of the invention comprising inhibitors of plexin-B1 and plexin-B2 is highly desirable and in particular useful for the medical treatments described in more detail herein below.

In accordance with a preferred embodiment of the first aspect of the invention the composition is a pharmaceutical composition.

In the pharmaceutical composition of the invention the compounds inhibiting the expression and/or the activity of the nucleic acid molecule and/or the protein according to the invention are preferably admixed with a pharmaceutically acceptable carrier or excipient to form a pharmaceutical composition. Suitable pharmaceutically acceptable carriers or excipients as well as the formulation of pharmaceutical compositions will be discussed herein below.

Via the pharmaceutical composition of the invention the compounds inhibiting the expression and/or the activity of the nucleic acid molecule and/or the protein according to the invention can be administered to a subject at a suitable dose and/or a therapeutically effective amount. Also this will be further discussed herein below. The length of treatment needed to observe changes and the interval following treatment for responses to occur vary depending on the desired effect. The particular amounts may be determined by conventional tests which are well known to the person skilled in the art. Suitable tests are, for example, described in Tamhane and Logan (2002), "Multiple Test Procedures for Identifying the Minimum Effective and Maximum Safe Doses of a Drug", Journal of the American statistical association, 97(457):1-9.

In accordance with the present invention, the term "pharmaceutical composition" relates to a composition for administration to a patient, preferably a human patient. The pharmaceutical composition of the invention comprises the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day. Furthermore, if for example said compound is an iRNA agent, such as an siRNA, the total pharmaceutically effective amount of pharmaceutical composition administered will typically be less than about 75 mg per kg of body weight, such as for example less than about 70, 60, 50, 40, 30, 20, 10, 5, 2, 1, 0.5, 0.1, 0.05, 0.01, 0.005, 0.001, or 0.0005 mg per kg of body weight. More preferably, the amount will be less than 2000 nmol of iRNA agent (e.g., about 4.4 x 10¹⁶ copies) per kg of body weight, such as for example less than 1500, 750, 300, 150, 75, 15, 7.5, 1.5, 0.75, 0.15, 0.075, 0.015, 0.0075, 0.0015, 0.00075 or 0.00015 nmol of iRNA agent per kg of body weight. The length of treatment needed to observe changes and the interval following treatment for responses to occur vary depending on the desired effect.

As will be further detailed herein below, the pharmaceutical composition of the invention can be used, for example, to treat or prevent cancer and bone diseases. However, the pharmaceutical composition may also be used for the treatment or prevention of further diseases.

The present invention relates in a second aspect to an inhibitor of plexin-B1 and an inhibitor of plexin-B2 as defined in connection with the first aspect of the invention for use in the treatment or prevention of a disease.

The definitions and preferred embodiments of the first aspect of the invention apply mutatis mutandis to the second aspect of the invention as far as being applicable to this second aspect. For instance, also in the context of the second aspect the inhibitor of plexin-B1 and the inhibitor of plexin-B2 may be formulated as a pharmaceutical composition as described above.

As used herein the term "disease" encompasses any particular abnormal condition that negatively affects the structure or function of part or all of an organism including an external injury, such a bone fracture. The organism is preferably human. A disease may be caused by external factors such as pathogens or by internal dysfunctions, thereby giving raise to diseases such as cancer.

In accordance with a preferred embodiment of the second aspect of the invention the disease is cancer.

Cancer is an abnormal malignant new growth of tissue that possesses no physiological function and arises from uncontrolled usually rapid cellular proliferation.

The cancer can be selected from the group consisting of breast cancer, ovarian cancer, endometrial cancer, vaginal cancer, vulvacancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, lymphomas and leukemias.

The cancer is preferably a solid tumor or cancer. A solid tumor or cancer is an abnormal mass of tissue that usually does not contain cysts or liquid areas by contrast to a liquid tumor.

In accordance with a more preferred embodiment of the second aspect of the invention the cancer is a colon cancer, gastrointestinal cancer, cervical cancer, ovarian cancer or bone cancer.

Colon cancer (also commonly referred to as colorectal cancer) is cancer of the large intestine (colon), which is the final part of the digestive tract. Colon cancer has a high incidence. In 2017, nearly 136,000 new cases of colorectal cancer were diagnosed in the U.S. About 1 in 20 (5%) Americans will develop colorectal cancer during their lifetime.

Gastrointestinal cancer refers to malignant conditions of the gastrointestinal tract (GI tract) and accessory organs of digestion, including the esophagus, stomach, biliary system, pancreas, small intestine, large intestine, rectum and anus. Esophageal cancer is the sixth-most-common cancer in the world, and its incidence is increasing.

Bone cancers are broken down into separate types based on the type of cell where the cancer began. The most common types and thus preferred types of bone cancer are chondrosarcoma, Ewing sarcoma and osteosarcoma. Chondrosarcoma is the second most common form of bone cancer. In this tumor, the cancerous cells produce cartilage. Chondrosarcoma usually occurs in the pelvis, legs or arms in middle-aged and older adults. Ewing sarcoma tumors most commonly arise in the pelvis, legs or arms of children and young adults. Osteosarcoma is the most common form of bone cancer. In this tumor, the cancerous cells produce bone. This variety of bone cancer occurs most often in children and young adults, in the bones of the leg or arm. In rare circumstances, osteosarcomas can arise outside of bones (extraskeletal osteosarcomas).

Cervical cancer develops in a woman's cervix (the entrance to the womb from the vagina). It mainly affects sexually active women aged between 30 and 45. This is because various strains of the human papillomavirus (HPV), a sexually transmitted infection, play a role in causing most cervical cancer.

Ovarian cancer occurs due to abnormal and uncontrolled cell growth in the ovaries. Most cases of ovarian cancer occur sporadically in subjects with little to no family history of the condition. However, approximately 10-25% of ovarian cancers are thought to be "hereditary."

Among colon cancer, gastrointestinal cancer, cervical cancer, ovarian cancer and bone cancer colon cancer is preferred.

In accordance with a further preferred embodiment of the second aspect of the invention the disease is a bone disease.

Bone disease refers to the medical conditions which affect the bone. An example is bone cancer as described herein above. In bone diseases bones generally break easily. Different kinds of bone problems include low bone density and osteoporosis, which make the bones weak and more likely to break, osteogenesis imperfecta which makes the bones brittle, Paget's disease which makes the bones weak. Bones can also develop infections. Other bone diseases are caused by poor nutrition, genetics, or problems with the rate of bone growth or rebuilding. All these bone diseases will benefit from osteogenesis, so that the bones can become stronger again.

In accordance with a more preferred embodiment of the second aspect of the invention the bone disease is associated with bone loss.

A disease is associated with bone loss is a disease being characterized by a decrease in bone mass and/or density. Means for determining bone mass and/or density are art established. For example, Quantitative computed tomography (QCT) is a medical technique that measures bone mineral density (BMD) using a standard X-ray Computed Tomography (CT) scanner with a calibration standard to convert Hounsfield Units (HU) of the CT image to bone mineral density values. Quantitative CT scans are primarily used to evaluate bone mineral density at the lumbar spine and hip.

In accordance with an even more preferred embodiment of the second aspect of the invention the bone disease being associated with bone loss is osteoporosis or periodontosis.

Osteoporosis is a disease that weakens bones to the point where they break easily-most often, bones in the hip, backbone (spine), and wrist. Osteoporosis is called a "silent disease" because affected individuals may not notice any changes until a bone breaks. All the while, though, bones may beve losing strength for many years. It is the most common reason for a broken bone among the elderly. Osteoporosis has a prevalence of about 30% in postmenopausal women and is a major risk factor for bone fractures, reduced quality of life and immobilization.

Periodontosis (or peridontal disease) refers to the loos of aveolar bone, preferably with inflammation. It may lead to the loss of teeth.

Osteoporosis is preferred over periodontosis.

In accordance with another more preferred embodiment of the second aspect of the invention the bone disease is a bone fracture.

A bone fracture is a medical condition in which there is a partial or complete break in the continuity of the bone. As discussed above, a bone fracture may be the result or may become more likely by certain bone diseases. However, also in the absence of such diseases a bone may break due to an accident of physical strength. Thus, a bone fracture may be the result of high force impact or stress, or a minimal trauma injury as a result of certain medical conditions that weaken the bones, where the fracture may then be termed a pathologic fracture.

The present invention relates in a third aspect to a method for engineering bone comprising culturing pluri- or multipotent stem cells under conditions that mediate bone formation, wherein the conditions comprise the inhibitor of plexin-B1 and the inhibitor of plexin-B2 as defined in connection with the first aspect of the invention.

The definitions and preferred embodiments of the first and second aspect of the invention apply mutatis mutandis to the third aspect of the invention as far as being applicable to this third aspect.

Methods for culturing pluri- or multipotent stem cells under conditions that mediate bone formation are known in the art. Cellular sources of bone have been identified in bone marrow, periosteum, skeletal muscle, fat, and umbilical cord blood (Walmsey (2016), Stem Cell Rev., 12(5):524-529). Preferably bone marrow-derived mesenchymal stem cells (MSCs) are used as the stem cells. MSCs are among the longest-studied of all stem cell populations, and most osteogenic tissue engineering strategies use MSCs as the starting cell population.

The conditions that mediate bone formation generally require that the pluri- or multipotent stem cells are cultured in the presence of certain growth factors (GFs) which drive the differentiation of the pluri- or multipotent stem cells into bone cells. The main families of GFs involved in bone regeneration include fibroblast GFs (FGF), bone morphogenetic proteins (BMPs), vascular endothelial GF (VEGF), insulin-like GF (IGF) and transforming GF β (TGFβ). Particular attention has been directed toward the use of BMP-2 and BMP-7, which have been incorporated in Food and drug Administration (FDA)-approved devices for bone regeneration. The GFs used in bone tissue engineering can be classified as inflammatory GFs and cytokines, pro-osteogenic GFs and angiogenic GFs (De Witte et al. (2018), Regen Biomater.; 5(4): 197-211). Also the inhibition of plexin-B1 and plexin-B2 has a pro-osteogenic effect.

In the claimed method stem cells are preferably cultured together with biomaterial scaffolds for the constructing of bone (Willerth et al., Combining stem cells and biomaterial scaffolds for constructing tissues and cell deliver, Stembook. Cambridge MA: Harvard Stem Cell Institute; 2008). In this context, a scaffold is an implantable material, either natural or synthetic, that provides appropriate support to the developing bone.

The scaffold can be thought of as a delivery vehicle for a seed population that contains stem cells and as a structural support for the burgeoning tissue that forms as the cells differentiate. To encourage tissue formation, the stem cells are attached to the scaffold and transplanted immediately, sometimes in conjunction with appropriate tissue growth factors. Choice of scaffolding depends on application, as materials must be compatible with the stem cell population and the tissue that is being regenerated. Ideally, a scaffold will: 1) enable the stem cells to retain critical characteristics such as the ability to self-renew; 2) allow the cells to differentiate appropriately; 3) provide adequate support for the developing tissue; 4) conform to the mechanical specifications of the injury site; and 5) ultimately be resorbed into the body without generating toxic by-products.

Researchers have investigated a wide variety of natural and synthetic materials and composite mixtures of materials as scaffolds for bone regeneration. Natural proteins such as collagen, fibrin, silk, and polysaccharides (chains of sugar molecules) such as hyaluronic acid, and chitosan have demonstrated potential as components of bone scaffolding. These materials offer the advantages of biocompatibility, biodegradability, limited toxicity, and the ability to be molded to meet the mechanical requirements of bone.

In accordance with another more preferred embodiment of the third aspect of the invention the method is an *ex vivo* or *in vitro* method.

An *ex vivo* method is not practiced on the human or animal body. *In vitro* (meaning: in the glass) methods are performed with microorganisms, isolated cells, or biological molecules outside their normal biological context.

In accordance with another more preferred embodiment of all the aspects of the invention the inhibitor of plexin-B1 and the inhibitor of plexin-B2 are (i) two distinct compounds, (ii) a bispecific compound inhibiting plexin-B1 and plexin-B2, or (iii) a compound inhibiting both plexin-B1 and plexin-B2.

Among options (i) to (iii), options (i) and (ii) are preferred. In that regard it is further preferred that in case of (i) one of the two compounds specifically inhibits plexin-B1 whereas the other compound specifically inhibits plexin-B2. In that respect, it is understood that no cross-reactivity occurs. It is also preferred that the two inhibitory entities of the bispecific compounds of (ii) are specific for either of the two plexins.

As the two distinct compounds two of the inhibitors as described in connection with the first aspect of the invention may be used, such as a first antibody specifically binding to plexin-B1 and an antibody specifically binding to plexin-B2. Also different types/classes may be used for the differential inhibition, such as an antibody for plexin-B1 and an siRNA for plexin-B2.

A bispecific compound is a single compound with two different binding entities, one specifically binding to plexin-B1 and one specifically binding to plexin-B2. The bispecific compound may be a bispecific antibody or antibody fragment. A minimalistic bispecific antibody is composed of the antigen-binding sites of two antibodies. The single-chain Fv (scFv) format is the most commonly used derivative of the VH and VL domains representing the minimal antigen-binding site of an antibody. Due to the single-chain configuration, bispecific antibodies can be built by connecting two scFvs through a linker (connector). Rather than connecting antigen-binding sites in a tandem arrangement, single-domain antibodies, such as VH or VL domains, VHH, VNAR and nanobodies, can be used to make bispecific molecules, and, in general this approach can also be applied to scaffold proteins, an emerging class of antibody mimetics. Several examples of classes of antibody mimetics are described herein above. They may be fused to single-antigen specific antibodies or fragments thereof to generate bispecific antibody constructs. Diabodies (Db) are bivalent molecules composed of two chains, each comprising a VH and VL domain, either from the same or from different antibodies. In the diabody format, the two variable domains are connected by a short linker that is usually 5 residues, e.g., GGGGS. Because the linker length is substantially shorter than that required to allow intrachain assembly of an antigen-binding site, which would result in a scFv, two chains dimerize in a head-to-tail orientation resulting in a compact molecule with a molecular mass similar to tandem scFv (∼50 kDa). Fc-less bispecific antibodies were obtained using Fabs as the building block to which additional binding units are fused. Fabs are heterodimeric molecules composed of a light chain and a heavy chain fragment (Fd) and can thus be used to generate bivalent, bispecific molecules, but also trivalent, bi- or trispecific fusion proteins, e.g., by fusing a scFv to the C-terminus of either the light chain or Fd (bibody Fab-L-scFv, Fab-H-scFv), or to both chains (tribody, Fab-(scFv)₂) (Brinkmann and Kontermann (2017), MAbs. 2017 Feb-Mar; 9(2):182-212). Similar constructs may be produced fro antibody mimetics discussed above.

Also nucleic acid constructs may be bispecfic. A non-limiting example is an expression vector expressing a siRNA aganist plexin-B1 and second siRNA against pelxin-B2.

As discussed herein above, plexin-B1 and plexin-B2 are highly homologous transmembrane receptors. Thus, for example, a homologous epitope of human plexin-B1 and plexin-B2 protein may be used to generate an antibody or ribozyme specifically binding to human plexin-B1 and plexin-B2 but not to other proteins, including other plexins. Likewise, for example, a homologous nucleotide stretch of human plexin-B1 and plexin-B2 protein may be used to generate an siRNA, shRNA or antisense construct specifically binding to the mRNA encoding Plexin-B1 and Plexin-B2 but not to other the mRNA of other proteins, including other plexins.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.

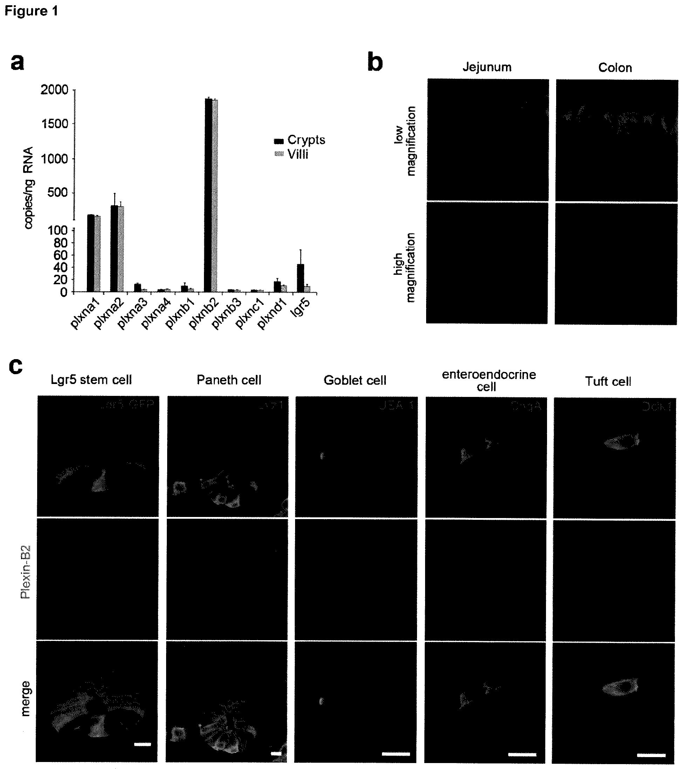

**Figure 1****: Expression of Plexin-B2 in the intestinal epithelium**. (**a**) From the mucosa of the jejunum, two fractions were isolated: one fraction contained mainly crypts, the other mainly villi. mRNA expression of plexins was determined by quantitative RT-PCR. (**b**) Immunstaining of Plexin-B2 (red). (c) Immunostaining for Plexin-B2 (red) on jejunum tissue sections of Lgr5 reporter mice, which express GFP under the control of the Lgr5 promoter (green; "Lgr5-GFP"; column on the very left), and double immunostainigs for Plexin-B2 (red) and cell type-specific marker proteins (green) on jejunum tissue sections of wildtype mice.
**Figure 2****: Generation and analysis of intestinal epithelium-specific Plexin-B1/Plexin-B2-double-knockout mice**. (**a**) Immunostainings for Plexin-B2 (red). (**b**) Quantitative RT-PCR for Plexin-B1 in the jejunum. (**c**) H&E-staining of tissue sections of the intestine ("Swiss Roll"). (**d**) Quantitative analysis of villus area and width. (**e-h**) Phenomaster analysis of (e) respiratory ratio, (f) food intake, (g) water intake, (h) physical activity (distance). (**i**) Oral glucose tolerance test. (**j**) 4 hours after oral administration of FITC-Dextran, FITC-Dextran was quantified in the serum by photometry. "Plexin-B1/- B2 DKO": Plexin-B1/Plexin-B2-double-knockout.

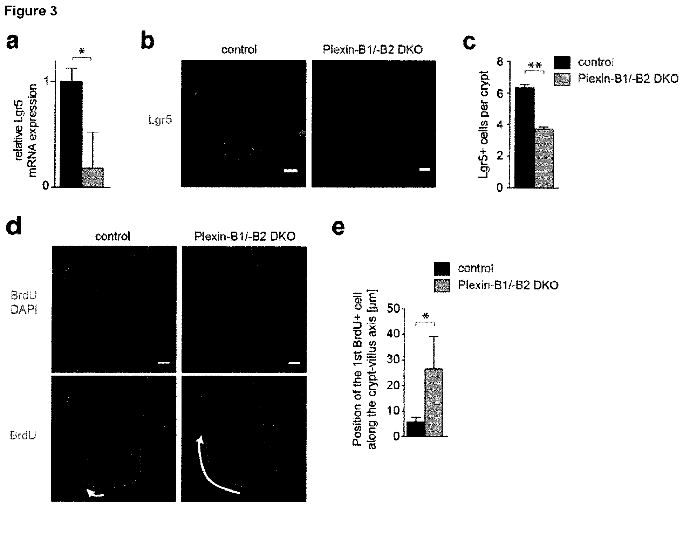

**Figure 3****: Plexin-B1/-B2-double-deficient mice display a reduced number and proliferation of intestinal stem cells.** (**a**) Quantitative RT-PCR for Lgr5 in the jejunum. (**b**) In-situ hybridization for Lgr5 in the mouse jejunum. (**c**) Quantification of the data in (b). (**d**) Anti-BrdU immunostaining 24 hours after BrdU injection. (**e**) Quantification of the distance between the crypt base and the first BrdU-positive cell along the crypt-villus axis (arrow).

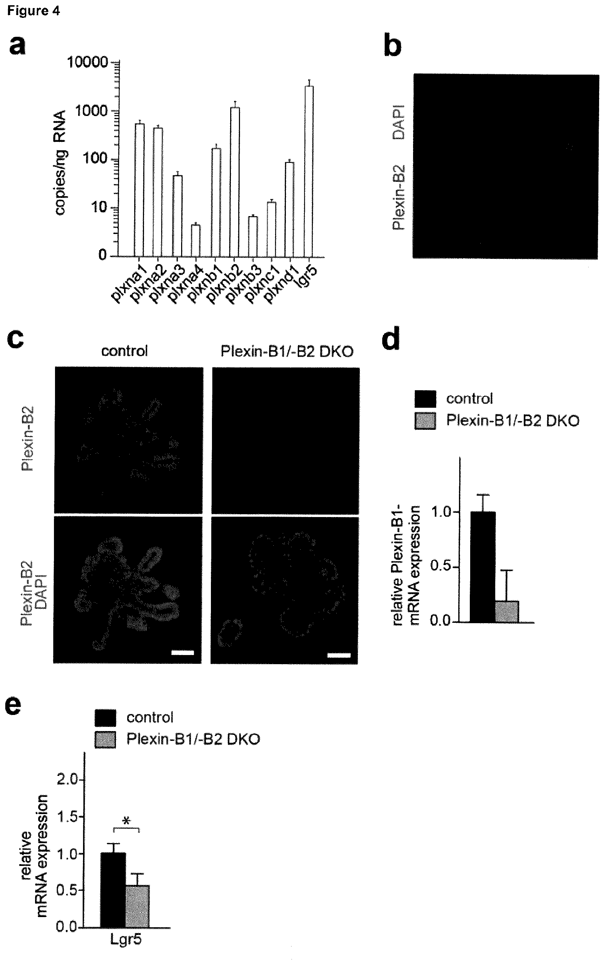

**Figure 4****: Plexin-B1 and Plexin-B2 regulate Lgr5 stem cells in murine intestinal organoids.** (**a**) Quantitative RT-PCR for plexins in intestinal murine organoids. (**b**) Immunostaining for Plexin-B2 (red) in intestinal organoid. (**c**) Immunostaining for Plexin-B2 (red) in control and Plexin-B1/Plexin-B2-double-knockout organoids. (**d**, **e**) Quantitative RT-PCR for (d) Plexin-B1 or (e) Lgr5 in control and Plexin-B1/Plexin-B2-double-knockout organoids.
**Figure 5****: The inactivation of Plexin-B1 and Plexin-B2 inhibits the formation of tumors in a mouse model of colorectal tumorigenesis.** Quantification of the number of tumor-bearing mice in Plexin-B1/Plexin-B2-double-deficient mice (left; PB1/PB2 DKO) and in Plexin-B2-single-deficient mice (right; PB2 SKO).
**Figure 6****: Expression of plexins in human intestinal tissue.** (**a**) Quantitative RT-PCR for plexins in normal human colon epithelium and in colon adenocarcinomas.

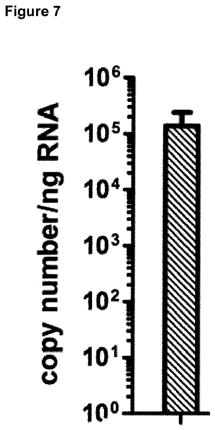

**Figure 7****. Plexin-B2 is expressed by primary murine osteoblasts.** Quantitative PCR for Plexin-B2 mRNA in primary murine osteoblasts.

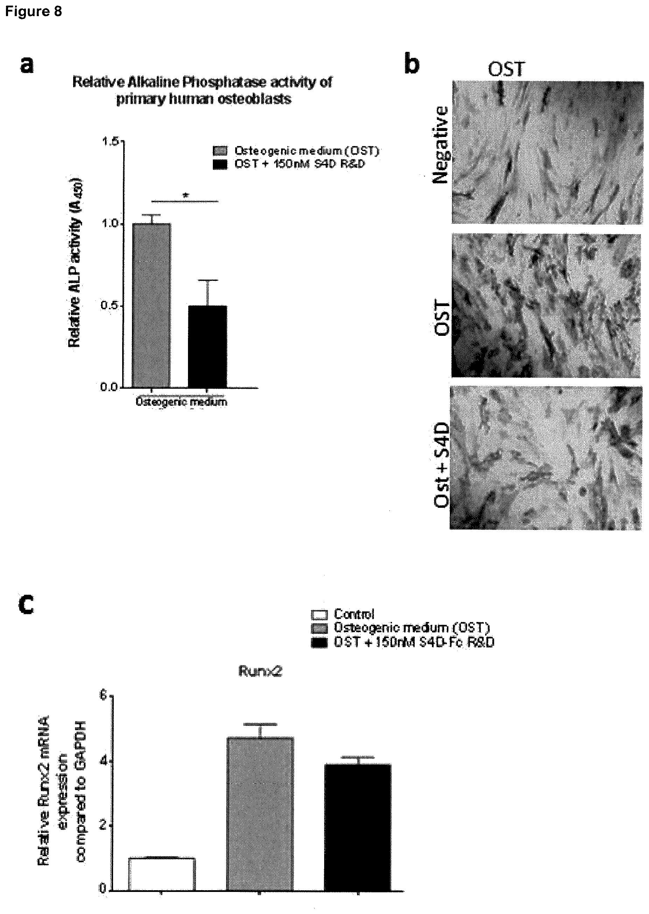

**Figure 8****. Activation of Plexin-B1/Plexin-B2 inhibits differentiation of osteoblasts. (a)** Application of Sema4D (150 nM) to primary human osteoblasts *in vitro* inhibits differentiation of osteoblasts (measured by activity of alkaline phosphatase, ALP). **(b)** Representative pictures of the quantification in (a). **(c)** Also the expression of Runx2, a marker for osteoblast differentiation, is inhibited by application of Sema4D.

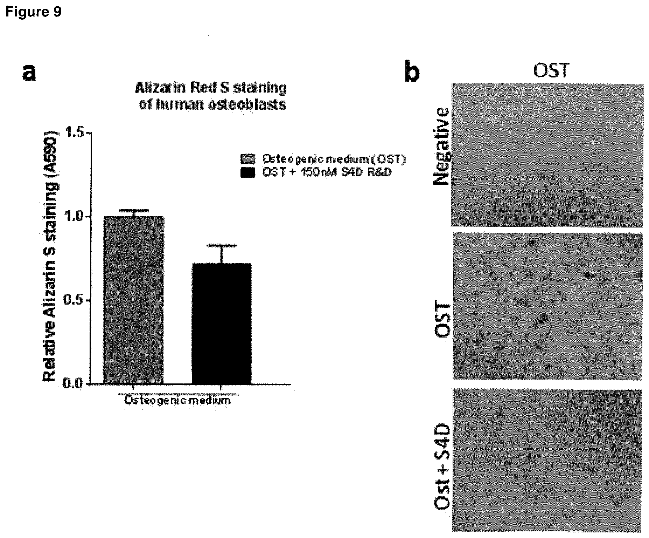

**Figure 9****. Activation of Plexin-B1/Plexin-B2 inhibits mineralization of osteoblasts. (a)** Application of Sema4D (150 nM) to primary human osteoblasts *in vitro* inhibits mineralization of osteoblasts (measured by Alizarin Red S staining). **(b)** Representative pictures of the quantification in (a).

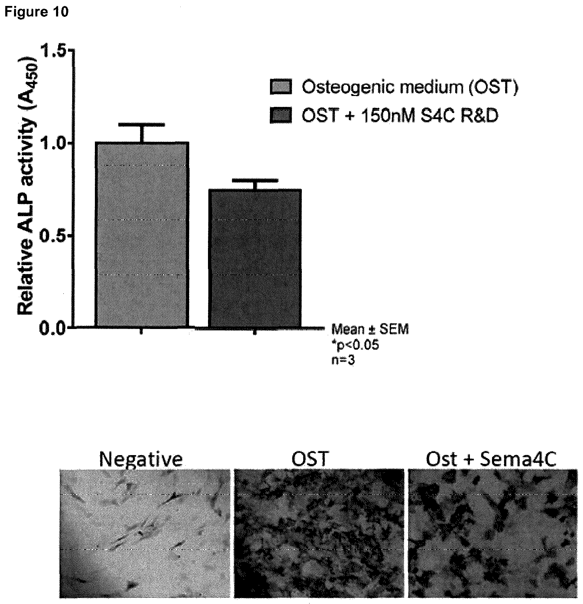

**Figure 10****. Activation of Plexin-B2 inhibits osteoblast function. (a)** Application of Sema4C (150 nM) to primary murine osteoblasts *in vitro* inhibits differentiation of osteoblasts (measured by the activity of alkaline phosphatase, ALP). **(b)** Representative pictures of the quantification in (a).

The examples illustrate the invention.

### Example 1- Plexin-B1 and Plexin-B2 in Colorectal Cancer

Colorectal cancer is one of the most prevalent cancers. Intestinal stem cells, which express the marker gene Lgr5, are centrally involved in the pathogenesis of colorectal cancer. In genetic mouse models (Kozar et al., 2013; Schepers et al., 2012) as well by employing human intestinal organoids from colorectal cancer patients (Cortina et al., 2017; Shimokawa et al., 2017) it was shown that neoplasias of the intestine are hierarchially organized, and that Lg5-positive cells act as cancer stem cells. Ablation of Lgr5 cells in murine or human colorectal cancer organoids leads to a growth arrest (Shimokawa et al., 2017, de Sousa e Melo et al., 2017). However, a therapeutic approach for colorectal cancer targeting cancer stem cells is currently not yet available.

Systematic mRNA expression analyses show that Plexin-B2 is the most highly expressed plexin of the intestinal mucosa (Fig. 1a). Also on the protein level, a strong expression of Plexin-B2 in the epithelium of the small and large intestine could be observed (Fig. 1b). Co-immunostainings for Plexin-B2 and cell type-specific marker proteins, as well as immunostainings for Plexin-B2 on tissue of Lgr5 reporter mice revealed that Plexin-B2 is expressed in Lgr5-positive stem cells (Fig. 1c). Plexin-B1 is enriched in Lgr5-positive intestinal stem cells (Munoz et al., 2012).

Mice with intestinal epithelium-specific inactivation of the genes encoding Plexin-B1 und Plexin-B2 showed no significant morphological, metabolic or other functional abnormalities under physiological conditions (Fig. 2).

Plexin-B1/Plexin-B2-double-deficient mice show a reduced mRNA expression of the stem cell-specific marker gene Lgr5 (Fig. 3a), a reduction of the number of Lgr5-positive stem cells (Fig. 3b und c) and a reduced proliferation of Lgr5 stem cells (Fig. 3d und e).

Under physiological conditions, Lgr5-positive stem cells are not essential for homeostasis of the intestinal epithelium: an ablation of Lgr5-positive stem cells can be compensated for by other (reserve) stem cell populations or by dedifferentiation of differentiated intestinal epithelial cells (Tetteh et al., 2016; Tian et al., 2011). For the progression of colorectal cancer, however, Lgr5-positive stem cells are essential (see above).

In intestinal organoids, which recapitulate the physiology and the functionality of the intestinal epithelium *in vivo* to a large extent (Sato et al., 2009), our data show that Plexin-B2 - exactly like in the intestinal epithelium *in vivo* - is the most highly expressed plexin (Fig. 4a und b). Plexin-B1 is also expressed in intestinal organoids (Fig. 4a). Consistent with the findings in the intestinal epithelium of Plexin-B1/-B2-knockout mice *in vivo*, Plexin-B1/-Plexin-B2-double-deficient organoids (Fig. 4c und d), have a reduced expression of Lgr5 (Fig. 4e). These results show that Plexin-B1 and Plexin-B2 exert an epithel-intrinsic function independently of non-epithelial intestinal cells.

In a genetic mouse model of colorectal cancer (Apc^{min} mutation), Plexin-B1/Plexin-B2-double knockout mice bear tumors much less frequently than respective control mice (Fig. 5, left). The reduction of tumor formation is less strong in Plexin-B2 single-deficient mice as compared to Plexin-B1/Plexin-B2-double-deficient mice (Fig. 5, right).

Also in the human intestine, both in normal as well as in tumor tissue, Plexin-B1 and Plexin-B2 are the most highly expressed plexins (Fig. 6).

Thus, Plexin-B1 and Plexin-B2 play a central role for the function of Lgr5-positive stem cells. Under physiological conditions, an inhibition of Plexin-B1 and Plexin-B2 function in the intestinal epithelium is compensated and stays functionally irrelevant. In tumors, however, a dual inhibition of the function of Plexin-B1 and Plexin-B2 leads to a significant reduction in tumor formation. In contrast to all other available therapeutic approaches, this strategy targets the function of cancer stem cells.

### Example 2 - Plexin-B1 and Plexin-B2 in Osteoperosis

Osteoporosis has a prevalence of around 30% among postmenopausal women, and represents a major risk factor for bone fractures, reduced life quality and immobilization. The currently approved drugs comprise estrogens and selective estrogen receptor modulators, bisphosphonates, the anti-RANKL antibody Denosumab, Strontium and parathyroid hormone (including its analogue Teriparatide). Despite of progress in the pharmacotherapy of osteoporosis, treatment options are still clearly insufficient and leave room for novel approaches with significant additional benefit.

Plexin-B1 (Dacquin et al., 2011; Negishi-Koga et al., 2011) and Plexin-B2 (Fig. 7) are both highly expressed on osteoblasts. A ligand for Plexin-B1 and Plexin-B2, Sema4D, is strongly expressed on osteoclasts (Dacquin et al., 2011; Negishi-Koga et al., 2011). Binding of Sema4D to Plexin-B1 results in the activation of the small GTPase RhoA and an inhibition of bone formation. Plexin-B1-deficient and Sema4D-deficient mice, and mice that express dominant-negative RhoA specifically in osteoblasts, display increased bone formation (Dacquin et al., 2011; Negishi-Koga et al., 2011). An anti-Sema4D antibody, which blocks binding of Sema4D to Plexin-B1, prevents bone loss in a mouse model for postmenopausal osteoporosis (Negishi-Koga et al., 2011).

Sema4D, which binds and activates both Plexin-B1 as well as Plexin-B2, inhibits the differentiation (Fig. 8) and mineralization (Fig. 9) of primary human osteoblasts in vitro.

Likewise, incubation of primary murine osteoblasts with Semaphorin 4C (Sema4C), a Plexin-B2-selective ligand, inhibits the differentiation of osteoblasts (Fig. 10).

The dual inhibition of Plexin-B1 and Plexin-B2, e.g. by inhibition of the binding of Plexin-B1/Plexin-B2 ligands, blocks the inhibitory effect of Plexin-B1 and Plexin-B2 on osteoblast differentiation, thereby counteracting osteoporosis development. Importantly and in contrast to most approved anti-osteoporotic drugs, this therapeutic principle relies on the enhancement of bone formation rather than on an inhibition of bone resorption.

### References

Cortina, C., Turon, G., Stork, D., Hernando-Momblona, X., Sevillano, M., Aguilera, M., Tosi, S., Merlos-Suárez, A., Stephan-Otto Attolini, C., Sancho, E., Batlle, E. A genome editing approach to study cancer stem cells in human tumors. EMBO Molecular Medicine 9:869-879 (2017)
Dacquin, R., Domenget, C., Kumanogoh, A., Kikutani, H., Jurdic, P., and Machuca-Gayet, I. 2011. Control of bone resorption by semaphorin 4D is dependent on ovarian function. PLoS One 6:e26627. Daviaud, N., Chen, K., Huang, Y., Friedel, R.H., Zou, H. Impaired cortical neurogenesis in plexin-B1 and -B2 double deletion mutant. Developmental Neurobiology 76:882-899 (2016)
de Sousa e Melo, F., Kurtova, A.V., Harnoss, J.M., Kljavin, N., Hoeck, J.D., Hung, J., Anderson, J.E., Storm, E.E., Modrusan, Z., Koeppen, H., Dijkgraaf, G.J., Piskol, R., de Sauvage, F.J. A distinct role for Lgr5+ stem cells in primary and metastatic colon cancer. Nature 543:676-680 (2017)
Hota PK, and Buck M. Plexin structures are coming: opportunities for multilevel investigations of semaphorin guidance receptors, their cell signaling mechanisms, and functions. Cell Mol Life Sci. 2012;69(22):3765-805.
Janssen BJ, Robinson RA, Perez-Branguli F, Bell CH, Mitchell KJ, Siebold C, et al. Structural basis of semaphorin-plexin signalling. Nature. 2010;467(7319):1118-22.
Jongbloets BC, Pasterkamp RJ. Semaphorin signaling during development. Development. 2014; 141(17):3292-7.
Kozar, S., Morrissey, E., Nicholson, A.M., van der Heijden, M., Zecchini, H.I., Kemp, R., Tavaré, S., Vermeulen, L., Winton, D.J. Continuous clonal labeling reveals small numbers of functional stem cells in intestinal crypts and adenomas. Cell Stem Cell 13:626-633 (2013)
Liu H, Juo ZS, Shim AH, Focia PJ, Chen X, Garcia KC, et al. Structural basis of semaphorin-plexin recognition and viral mimicry from Sema7A and A39R complexes with PlexinC1. Cell. 2010;142(5):749-61.
Muñoz, J., Stange, D.E., Schepers, A.G., van de Wetering, M., Koo, B.K., Itzkovitz, S., Volckmann, R., Kung, K.S., Koster, J., Radulescu, S., Myant, K., Versteeg, R., Sansom, O.J., van Es, J.H., Barker, N., van Oudenaarden, A., Mohammed, S., Heck, A.J., Clevers, H. The Lgr5 intestinal stem cell signature: robust expression of proposed quiescent '+4' cell markers. EMBO Journal 31:3079-3091 (2012)
Negishi-Koga, T., Shinohara, M., Komatsu, N., Bito, H., Kodama, T., Friedel, R.H., and Takayanagi, H. 2011. Suppression of bone formation by osteoclastic expression of semaphorin 4D. Nat Med 17:1473-1480.
Nogi T, Yasui N, Mihara E, Matsunaga Y, Noda M, Yamashita N, et al. Structural basis for semaphorin signalling through the plexin receptor. Nature. 2010;467(7319):1123-7.
Perälä, N., Jakobson, M., Ola, R., Fazzari, P., Penachioni, J.Y., Nymark, M., Tanninen, T., Immonen, T., Tamagnone, L., Sariola, H. Sema4C-Plexin B2 signalling modulates ureteric branching in developing kidney. Differentiation 81:81-91 (2011)
Sato, T., Vries, R.G., Snippert, H.J., van de Wetering, M., Barker, N., Stange, D.E., van Es, J.H., Abo, A., Kujala, P., Peters, P.J., Clevers, H. Single Lgr5 stem cells build crypt-villus structures in vitro without a mesenchymal niche. Nature 2009 459:262-265 (2009)
Schepers, A.G., Snippert, H.J., Stange, D.E., van den Born, M., van Es, J.H., van de Wetering, M., Clevers, H. Lineage tracing reveals Lgr5+ stem cell activity in mouse intestinal adenomas. Science 337:730-735 (2012)
Shimokawa, M., Ohta, Y., Nishikori, S., Matano, M., Takano, A., Fujii, M., Date, S., Sugimoto, S., Kanai, T., Sato, T. Visualization and targeting of LGR5+ human colon cancer stem cells. Nature 545:187-192 (2017)
Tetteh, P.W., Basak, O., Farin, H.F., Wiebrands, K., Kretzschmar, K., Begthel, H., van den Born, M., Korving, J., de Sauvage, F., van Es, J.H., van Oudenaarden, A., Clevers, H. Replacement of Lost Lgr5-Positive Stem Cells through Plasticity of Their Enterocyte-Lineage Daughters. Cell Stem Cell 18:203-213 (2016)
Tian, H., Biehs, B., Warming, S., Leong, K.G., Rangell, L., Klein, O.D., de Sauvage, F.J. A reserve stem cell population in small intestine renders Lgr5-positive cells dispensable. Nature 478:255-259 (2011)
Verlinden L, Vanderschueren D, Verstuyf A. Semaphorin signaling in bone. Mol Cell Endocrinol. 2016; 432:66-74.
Worzfeld T, Offermanns, S. Semaphorins and plexins as therapeutic targets. Nat Rev Drug Discov. 2014;13:603-621.
Xia, J., Swiercz, J.M., Bañón-Rodríguez, I., Matković, I., Federico, G., Sun, T., Franz, T., Brakebusch, C.H., Kumanogoh, A., Friedel, R.H., Martin-Belmonte, F., Gröne, H.J., Offermanns, S., Worzfeld, T. Semaphorin-Plexin Signaling Controls Mitotic Spindle Orientation during Epithelial Morphogenesis and Repair. Developmental Cell 33, 299-313 (2015)

## Claims

1. A composition comprising an inhibitor of plexin-B1 and an inhibitor of plexin-B2, wherein the inhibitor of plexin-B1 and/or the inhibitor of plexin-B2 is/are preferably selected from
(i) an inhibitor of the nucleic acid molecule encoding plexin-B1 and/or plexin-B2 selected from a small molecule, an aptamer, a siRNA, a shRNA, a miRNA, a morpholino, a ribozyme, an antisense nucleic acid molecule, a CRISPR-Cas9-based construct, a CRISPR-Cpf1-based construct, a meganuclease, a zinc finger nuclease, and a transcription activator-like (TAL) effector (TALE) nuclease, and/or
(ii) an inhibitor of the plexin-B1 and/or plexin-B2 protein selected from a small molecule, an antibody or antibody mimetic, an aptamer, wherein the antibody mimetic is preferably selected from affibodies, adnectins, anticalins, DARPins, avimers, nanofitins, affilins, Kunitz domain peptides and Fynomers®.

2. The composition of claim 1, wherein the composition is a pharmaceutical composition.

3. An inhibitor of plexin-B1 and an inhibitor of plexin-B2 as defined in claim 1 for use in the treatment or prevention of a disease.

4. The inhibitor of plexin-B1 and the inhibitor of plexin-B2 for use of claim 3, wherein the disease is cancer.

5. The inhibitor of plexin-B1 and the inhibitor of plexin-B2 for use of claim 4, wherein the cancer is a colon cancer, gastrointestinal cancer, cervical cancer, ovarian cancer or bone cancer.

6. The inhibitor of plexin-B1 and the inhibitor of plexin-B2 for use of claim 3, wherein the disease is bone disease.

7. The inhibitor of plexin-B1 and the inhibitor of plexin-B2 for use of claim 6, wherein the bone disease is associated with bone loss.

8. The inhibitor of plexin-B1 and the inhibitor of plexin-B2 for use of claim 7, wherein the bone disease being associated with bone loss is osteoporosis or periodontosis.

9. The inhibitor of plexin-B1 and the inhibitor of plexin-B2 for use of claim 6, wherein the bone disease is a bone fracture.

10. A method for engineering bone comprising culturing pluri- or multipotent stem cells under conditions that mediate bone formation, wherein the conditions comprise the inhibitor of plexin-B1 and the inhibitor of plexin-B2 as defined in claim 1.

11. The method of claim 10, wherein the method is an *ex vivo* or *in vitro* method.

12. The composition of claim 1 or 2; the inhibitor of plexin-B1 and the inhibitor of plexin-B2 for use of any one of claims 3 to 9; or the method of claim 10 or 11, wherein the inhibitor of plexin-B1 and the inhibitor of plexin-B2 are (i) two distinct compounds, (ii) a bispecific compound inhibiting Plexin-B1 and Plexin-B2, or (iii) a compound inhibiting both Plexin-B1 and Plexin-B2.
